(19) **Europäisches Patentamt / European Patent Office / Office européen des brevets**

(11) **EP 3 032 948 B1**

(12) **EUROPEAN PATENT SPECIFICATION**

(45) Date of publication and mention of the grant of the patent:
**25.12.2019 Bulletin 2019/52**

(21) Application number: **14835784.1**

(22) Date of filing: **12.08.2014**

(51) Int Cl.:
*A61K 9/20* (2006.01)    *A61K 31/485* (2006.01)
*B29C 43/48* (2006.01)    *B29K 105/00* (2006.01)
*B29K 71/00* (2006.01)    *B29C 43/46* (2006.01)
*B29C 48/00* (2019.01)

(86) International application number:
**PCT/US2014/050737**

(87) International publication number:
**WO 2015/023675 (19.02.2015 Gazette 2015/07)**

(54) **EXTRUDED IMMEDIATE RELEASE ABUSE DETERRENT PILL**

EXTRUDIERTE MISSBRAUCHSSICHERE PILLE MIT SOFORTIGER FREISETZUNG

COMPRIMÉ EXTRUDÉ ANTI-ABUS À LIBÉRATION IMMÉDIATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.08.2013 US 201361864926 P**
**16.04.2014 US 201461980242 P**

(43) Date of publication of application:
**22.06.2016 Bulletin 2016/25**

(73) Proprietor: **Pharmaceutical Manufacturing Research Services,
Inc.
Horsham, PA 19044 (US)**

(72) Inventors:
• **THOMPSON, Edwin, R.
Horsham, PA 19044 (US)**
• **THOMPSON, Eric, R.
Chalfont, PA 18914 (US)**
• **MYSLINSKI, Nicholas, R.
Bensalem, PA 19020 (US)**
• **KEMENY, Steven, F.
Philadelphia, PA 19128 (US)**

(74) Representative: **Mewburn Ellis LLP
City Tower
40 Basinghall Street
London EC2V 5DE (GB)**

(56) References cited:
US-A1- 2008 020 032    US-A1- 2009 004 267
US-A1- 2011 159 100    US-A1- 2011 159 100
US-A1- 2011 262 539    US-A1- 2012 065 220
US-A1- 2012 135 075    US-A1- 2013 028 970
US-A1- 2013 280 176

• Us Pharmacopeia: "USP <711> Dissolution Test Method", , 1 December 2011 (2011-12-01), XP055308776, Retrieved from the Internet: URL:http://www.usp.org/sites/default/files /usp_pdf/EN/USPNF/2011-02-25711DISSOLUTIO N .pdf [retrieved on 2016-10-07]

Remarks:
The file contains technical information submitted after the application was filed and not included in this specification

EP 3 032 948 B1

## Description

### Cross-Reference to Related Applications

This application claims priority to U.S. Provisional Application Nos. 61/864,926, filed August 12, 2013, and 61/980,242, filed April 16, 2014.

### Field of the Technology

The present disclosure relates to an oral immediate release, abuse deterrent pill prepared using a hot melt extrusion process and a forming unit. The pill contains at least one abuse deterrent mechanism to reduce abuse by non-oral administration routes, e.g. intranasal and/or intravenous. The extrusion process and forming unit are designed to efficiently prepare the abuse deterrent pill under conditions that reduce, or substantially eliminate, degradation of the active substance.

### Background

FDA-approved drugs are provided in many different forms based on the type of active substance, the indication treated and the preferred route of administration. These forms include enteral formulations (e.g., tablets, capsules or pills), parenteral formulations (e.g., injectable formulations such as intravenous, subcutaneous, intramuscular and intraarticular), liquid formulations (e.g., elixirs), lyophilized formulations and topical formulations. A majority of the FDA-approved drugs are currently available in enteral form, as either a tablet or capsule.

The production of pharmaceutical drugs in pill form by hot melt extrusion is relatively uncommon. While the idea of dissolving drugs in polymers and using extrusion to produce a pill has been known for decades, only a handful of FDA-approved drugs are extruded. Recently, extrusion techniques have been investigated for preparing abuse deterrent formulations. For example, U.S. 8,075,872 (assigned to Grunenthal GmbH) is directed to a thermoshaped abuse deterrent dosage form prepared with the assistance of an extruder. The extrudate may be shaped by the assistance of contra-rotating calendar rolls, and singulated by conventional means such as chopping. U.S. 8,383,152 (assigned to Grunenthal GmbH) is directed to a controlled release pharmaceutical dosage form that may also be prepared by extrusion and shaped by a calendaring process. U.S. 2007/0190142 (assigned to Abbott GmbH) is directed to a sustained release abuse deterrent dosage form prepared by extrusion and shaping the extrudate into a dosage form without a milling or multi-particulating step.

US 2013/0028970 A1 discloses a tamper-resistant tablet having a matrix material in an amount of more than one third of the total weight of the tablet, and a plurality of coated particulates in an amount of less than two thirds of the total weight of the tablet forming a discontinuous phase within the matrix material. The particulates comprise a pharmacologically active compound and a physiologically acceptable polymer. Also disclosed is a method of using the tablet to treat pain and other conditions.

US 2012/0065220 A1 discloses a pharmaceutical dosage form and method of use. The dosage form exhibits a breaking strength of at least 500 N and contains a pharmacologically active ingredient A, a physiologically acceptable polymer B obtainable by polymerization of a particular kind of monomer, and a polyalkylene oxide C with a weight average molecular weight of at least 200,000 g/mol. The content of C is at least 20wt% based on the total weight of the dosage form and A is present in a controlled-release matrix comprising B and C.

US 2011/0159100 A1 discloses controlled release formulations and methods of preparing them for delivery of active drug substances.

### Summary

The present disclosure relates to an abuse deterrent pill prepared using a hot melt extrusion process and a forming unit. The formulation contains an active substance susceptible to abuse and at least one abuse deterrent mechanism to reduce abuse by non-oral administration routes (e.g., intranasal and/or intravenous). The abuse deterrent pill is designed for immediate release of the active substance upon oral administration. The method of preparing the pill utilizes a hot melt extrusion process coupled with an in-line forming unit which eliminates the need for traditional extrusion processing steps, such as chopping the extrudate and molding the cut extrudate into a final form. The hot melt extrusion process and forming unit are operated under conditions that reduce, or substantially eliminate degradation of the active substance.

In one embodiment, the present disclosure relates to a directly-formed oral, extruded, immediate release, abuse deterrent pill comprising an active substance susceptible to abuse, a matrix agent and a plasticizer, wherein the pill is directly formed from a hot melt extrusion process, preferably without further processing (e.g., a cutting step). The extrudate may be re-sized or re-shaped, without cutting, prior to forming (e.g., rope sizer).

In another embodiment, the present disclosure relates to an oral, immediate release, abuse deterrent pill comprising an active substance susceptible to abuse, a matrix agent and a plasticizer, wherein the active substance susceptible to abuse has an immediate release profile, and wherein the composition includes a physical barrier to reduce abuse. In some embodiments, the physical barrier can reduce abuse by techniques such as pulverizing and swallowing, pulverizing and snorting, or pulverizing and injecting. In other embodiments, the physical barrier can reduce abuse by forming a hydrogel upon exposure to an aqueous or semi-aqueous solution. In some embodiments, the semi-aqueous solution is a 95% ethanol/5% water solution.

In another embodiment, the present disclosure relates to a process for the production of an oral, immediate release, abuse deterrent pill containing at least one active substance susceptible to abuse comprising processing a uniform blend of the at least one active substance susceptible to abuse, a matrix agent and a plasticizer by hot melt extrusion to form an extrudate. The extrudate may therein be formed into the pill using a forming unit.

In another embodiment, the present disclosure relates to a process for the production of an oral, immediate release, abuse deterrent pill containing at least one active substance susceptible to abuse comprising one or more of the following steps: combining the at least one active substance susceptible to abuse, a matrix agent and a plasticizer in a hopper to form a mixture; blending the mixture in the hopper until a uniform blend is achieved; monitoring the mixture during blending using a process analytical technique to determine when a uniform blend is achieved; feeding the uniform blend into an extruder; processing the uniform blend by hot melt extrusion to produce an extrudate; transferring the extrudate to a forming unit using a transfer unit capable of controlling the temperature, pressure, environment and/or shape of the extrudate; forming the extrudate using the forming unit into the pill; and determining the quality, volume and weight of the pill using an optical inspection technique.

In another embodiment, the present disclosure relates to a dosage form for use in a method of treating pain comprising administering to an individual in need thereof a therapeutically effective amount of an abuse deterrent formulation prepared using a hot melt extrusion process and a forming unit as described herein.

The invention concerns an oral, immediate release, abuse deterrent dosage form as defined in claim 1 and a process for its production according to claim 8.

Preferred features are set out in the dependent claims.

**Brief Description of the Drawings**

Figure 1 shows an embodiment of the extruder (14) and forming unit (60). The extruder has multiple temperature zones (e.g., 20-30) and pressure zones (e.g., 20, 40-43) to control the formation of a uniform extrudate under conditions that reduce, or substantially eliminate, degradation of the active substance.

Figure 2 shows an embodiment of a chain forming unit. The chain forming unit includes an upper and lower chain system (110 and 112) and tooling (100) to form the incoming extrudate (56) into formed pills (19).

Figure 3 shows an embodiment of an extrudate sizing apparatus (e.g., rope sizer). The rope sizer includes consecutive rollers (90-96) rotating at consecutively faster speeds for accepting an incoming extrudate (52) and expelling a faster moving, re-sized (smaller diameter) extrudate (54).

Figure 4 shows the percent release of the active substance (i.e., acetaminophen dissolution in 45 minutes) versus the weight percent of the matrix agent (e.g., polyethylene oxide or PEO) for two similar abuse deterrent pill formulations having different molecular weight matrix agents (e.g., 300K Daltons PEO vs. 600K Daltons PEO).

Figure 5 shows the weight percent of particles formed by grinding which have a particle size less than 0.5 mm versus the weight percent of matrix agent (e.g., PEO). The particles are formed by grinding the formulation in a commercial coffee grinder for at least 30 seconds.

Figure 6 shows equipment capable of executing traditional "tablet breaking force" analysis.

Figures 7 - 9 show equipment capable of executing a "cutting force" analysis including a fracture wedge set attachment used to mimic common kitchen scissors (Figures 7 and 8 showing different views) and a razor blade attachment (Figure 9).

Figure 10 shows cutting force data tables for the razor blade and the fracture wedge attachments.

Figures 11 and 12 show particle size analysis and grinding results for exemplary formulations.

Figure 13 shows the results of the dissolution, purity and dye evaluation tests on exemplary formulations.

**Detailed Description**

Abuse of prescription drugs, particularly opioids, is a serious and growing public health concern. To address this concern, new formulations are being developed that contain abuse-deterrent properties. Abuse deterrent properties include properties that make product manipulation more difficult or make abuse of the manipulated product less attractive or rewarding.

Recently the FDA issued a draft guidance for industry related to formulations having abuse deterrent properties. Guidance for Industry: Abuse-Deterrent Opioids - Evaluation and Labeling, U.S. Department of Health and Human Services, FDA, CDER, January 2013. These guidelines separate abuse deterrent formulations into six categories, including: physical/chemical barriers, agonist/antagonist combinations, aversion, delivery system, prodrug, or a combination of the aforementioned. As described by the FDA guidance, the categories are:

Physical/Chemical barriers - Physical barriers can prevent chewing, pulverizing, cutting, grating, or grinding. Chemical barriers can resist extraction of the opioid using common solvents like water, alcohol, or other organic solvents. Physical and chemical barriers can change the physical form of an oral drug rendering it less amenable to abuse.

Agonist/Antagonist combinations - An opioid antagonist can be added to interfere with, reduce, or defeat the euphoria associated with abuse. The antagonist can be sequestered and released only upon manipulation of the product. For example, a drug product may be formulated such that the substance that acts as an antagonist is not clinically active when the product is swallowed but becomes active if the product is crushed and injected or snorted.

Aversion - Substances can be combined to produce an unpleasant effect if the dosage form is manipulated prior to ingestion or a higher dosage than directed is used.

Delivery System (including depot injectable formulations and implants) - Certain drug release designs or the method of drug delivery can offer resistance to abuse. For example, a sustained-release depot injectable formulation that is administered intramuscularly or a subcutaneous implant can be more difficult to manipulate.

Prodrug - A prodrug that lacks opioid activity until transformed in the gastrointestinal tract can be unattractive for intravenous injection or intranasal routes of abuse.

Combination - Two or more of the above methods can be combined to deter abuse.

An opioid analgesic submitted for abuse deterrent formulation (ADF) labeling must show conformance to one or more of these categories. The present disclosure relates to an abuse deterrent pill for oral administration, which provides immediate release of an active pharmaceutical substance and conforms to one or more of these categories. In one embodiment, the abuse deterrent formulation of the present disclosure conforms to at least one of the six FDA categories. In another embodiment, the abuse deterrent formulation of the present disclosure conforms to at least two of the six FDA categories. In another embodiment, the abuse deterrent formulation of the present disclosure conforms to at least three of the six FDA categories. In another embodiment, the abuse deterrent formulation of the present disclosure conforms to at least four of the six FDA categories. In another embodiment, the abuse deterrent formulation of the present disclosure conforms to at least five of the six FDA categories.

For example, an abuse deterrent pill of the present disclosure can reduce abuse by the incorporation of at least one physical barrier. The physical barrier is designed to prevent abuse based on chewing, pulverizing, cutting, grating or grinding. Preferably, the physical barrier prevents or reduces the effectiveness of these methods. As used herein, the phrase "abuse deterrent" means that the active substance cannot readily be separated from the formulation in a form suitable for abuse by such means as, for example, grinding. The abuse deterrent pill of the present disclosure cannot be easily ground, extracted from, or both. Abuse deterrent measures render it difficult to transform the pill into a powder or extract for non-oral administration, such as intranasal or intravenous.

In one embodiment, the present disclosure relates to a directly-formed, extruded, oral, immediate release, abuse deterrent pill. The pill includes an active substance susceptible to abuse, a matrix agent and a plasticizer. After extrusion, the extrudate is directly formed into the pill without further processing, such as the use of a cutting step.

As used herein, the term "active substance" or "active substance susceptible to abuse" means an opioid or opioid related compound subject to potential abuse. The active substance may include, without limitation, alfentanil, allylprodine, alphaprodine, amphetamine, anileridine, benzylmorphine, bezitramide, buprenorphine, butorphanol, clonitazene, codeine, cyclazocine, desomorphine, dextroamphetamine, dextromoramide, dezocine, diampromide, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levallorphan, levophenacylmorphan, levorphanol, lofentanil, meperidine, meptazinol, metazocine, methadone, metopon, morphine, myrophine, nalbulphine, narceine, nicomorphine, norpipanone, opium, oxycodone, papvretum, pentazocine, phenadoxone, phenazocine, phenomorphan, phenoperidine, piminodine, propiram, propoxyphene, sufentanil, tilidine, and tramadol, and pharmaceutically acceptable salts and mixtures thereof. Preferably, the active substance is either oxycodone or hydrocodone. In one embodiment, the formulation of the present disclosure excludes oxymorphone. For example, the formulation of the present disclosure contains at least one active substance susceptible to abuse, provided the active substance is not oxymorphone.

The amount of active substance in the formulation may vary depending on the active substance, stability, release profile and bioavailability. The amount of active substance in the formulation may range from about 0.50 Wt% to about 15 Wt%. Particularly, the amount of active substance in the formulation may range from about 0.75 Wt% to about 14 Wt%, or from about 1.0 Wt% to about 13 Wt%, or from about 2.0 Wt% to about 12 Wt%, or from about 3.0 Wt% to about 11 Wt%, or from about 5.0 Wt% to about 10 Wt%. For example, the formulation may be a 100 mg pill having about 5 mg or about 10 mg of active substance (e.g., oxycodone HCl or hydrocodone bitartrate).

In another embodiment, the amount of active substance in the formulation may range from about 0.50 Wt% to about 40 Wt%. Particularly, the amount of active substance in the formulation may range from about 0.75 Wt% to about 37.5 Wt%, or from about 1.0 Wt% to about 35 Wt%, or from about 2.0 Wt% to about 34 Wt%, or from about 3.0 Wt% to about 32.5 Wt%, or from about 5.0 Wt% to about 30 Wt%. For example, the formulation may be a 100 mg pill having about 5 mg or about 30 mg of active substance (e.g., oxycodone HCl or hydrocodone bitartrate).

Formulations of the present disclosure may also include an additional active ingredient. Additional active ingredients include other analgesics, such as acetaminophen, ibuprofen, acetylsalicylic acid and/or naproxen. For example, the formulation may include an oxycodone HCl/acetaminophen combination, or a hydrocodone bitartrate/acetaminophen combination.

In one embodiment, the formulation includes at least one active substance, e.g. hydrocodone bitartrate, and at least one additional ingredient, e.g. acetaminophen. The amount of the at least one active substance and the additional ingredient in the formulation may vary depending on the active substance or the additional ingredient, their stability, their release profile and their bioavailability. In addition to the ranges provided above for the amount of active substance in the formulation, the amount of active substance in the formulation may also range from about 0.10 Wt% to about 10.0 Wt%. Particularly, the amount of active substance in the formulation may range from about 0.20 Wt% to about 8.0 Wt%, or from about 0.3 Wt% to about 6.0 Wt%, or from about 0.4 Wt% to about 4.0 Wt%, or from about 0.5 Wt% to about 2.0 Wt%. The amount of additional ingredient in the formulation may range from about 15 Wt% to about 80 Wt%. Particularly, the amount of additional substance in the formulation may range from about 20 Wt% to about 75 Wt%, or from about 25 Wt% to about 70 Wt%, or from about 30 Wt% to about 65 Wt%. For example, the formulation may be a 500 mg or 1,000 mg pill having about 5 mg or about 10 mg of active substance (e.g., oxycodone HCl or hydrocodone bitartrate) and about 300 mg or about 325 mg of additional analgesic (e.g. acetaminophen).

The dosage form of the present disclosure can be rendered abuse deterrent by incorporating at least one matrix agent in the formulation to increase the strength of the tablet beyond that of conventional dosage forms. The matrix agent increases the dosage form's resistance to physical or mechanical forces, such as pulverizing or grinding. By selecting the appropriate molecular weight grade and the quantity present within a formulation, the strength characteristics of the dosage form can be manipulated in a way to create a wide array of abuse deterrent pills have immediate release profiles.

The matrix agent may also render the dosage form abuse deterrent by acting as a gelling or viscosity increasing agent. Upon contact with a solvent (e.g., aqueous or semi-aqueous solution), the dosage form is capable of absorbing the solvent and swelling to form a viscous or semi-viscous substance. The formation of a viscous or semi-viscous substance significantly reduces and/or minimizes the amount of free solvent which can contain an amount of active substance, and which can be drawn into a syringe. The matrix agent can also reduce the overall amount of active substance extractable with the solvent by entrapping the active substance in a gel matrix. Typical matrix agents include pharmaceutically acceptable polymers, typically hydrophilic polymers, such as those that form hydrogels. These properties allow for an oral drug delivery system that satisfies at least one of the categories in the FDA guidance (e.g., "physical and chemical barriers can change the physical form of an oral drug rendering it less amenable to abuse").

The matrix agent may exhibit a high degree of viscosity upon contact with a suitable solvent. The high viscosity can enhance the formation of highly viscous gels when attempts are made to crush and dissolve the contents of a formulation in an aqueous or semi-aqueous vehicle and inject it intravenously. For example, when an abuser crushes and dissolves the formulation in a solvent, a viscous or semi-viscous gel is formed. The increase in the viscosity of the solution discourages the abuser from injecting the gel intravenously or intramuscularly by preventing the abuser from transferring sufficient amounts of the solution to a syringe.

Suitable matrix agents are natural or synthetic polymers capable of providing increased resistance to pulverizing or grinding. Disclosed matrix agents include agar, alamic acid, alginic acid, carmellose, carboxymethylcellulose sodium, chitosan, copovidone, dextrin, gelatin, hydroxyethyl cellulose, hydroxypropyl cellulose, hypromellose (HPMC), methylcellulose derivatives, microcrystalline cellulose, polyacrylic acid, polyalkalene oxide (e.g., polymethylene oxide, polyethylene oxide and polypropylene oxide), polyvinyl acetate, polyvinyl alcohol, povidone, propylene glycol alginate, polyvinylcaprolactam - polyvinyl acetate - polyethylene glycol graft co-polymer, pullulan, silicon dioxide, sodium alginate, starch, and vinylpyrrolidone-vinyl acetate copolymers. In claim 1, the matrix agent is a polyethylene oxide. Polyethylene oxide is a non-ionic, water soluble polymer that is readily available in a wide range of molecular weight grades.

The matrix agent should be capable of both ensuring the formation of a solid dosage form by extrusion and allowing immediate release of the active substance. The formulation of the present disclosure may accomplish both capabilities by using a matrix agent having an appropriate molecular weight (or appropriate average molecular weight), such as between about 50K Daltons and about 300K Daltons as disclosed herein.

In disclosed embodiments, the matrix agent has a molecular weight between about 100K and about 300K Daltons. Particularly, the matrix agent has a molecular weight between about 150K and about 250K Daltons, or about 180K and about 220K Daltons, or about 190K and about 210K Daltons, or about 195K and about 205K Daltons.

In other disclosed embodiments, the matrix agent has a molecular weight between about 100K and about 200K Daltons. Particularly, the matrix agent has a molecular weight between about 120K and about 180K Daltons, or about 130K and about 170K Daltons, or about 140K and about 160K Daltons, or about 145K and about 155K Daltons.

In claim 1, the matrix agent has a molecular weight between about 50K and about 150K Daltons. Particularly, the matrix agent has a molecular weight between about 80K and about 120K Daltons, or about 85K and about 115K Daltons, or about 90K and about 110K Daltons, or about 95K and about 105K Daltons.

In another embodiment, the matrix agent has a molecular weight between about 50K and about 100K Daltons. Particularly, the matrix agent has a molecular weight between about 55K and about 95K Daltons, or about 60K and about 90K Daltons, or about 65K and about 85K Daltons, or about 70K and about 80K Daltons.

The performance of the matrix agent and the formulation is also dependent on the amount of matrix agent present in the formulation. The formulation, or final dosage form, may contain about 10 Wt% to about 90 Wt% matrix agent as disclosed herein.

In disclosed embodiments, the formulation contains between about 40 Wt% and 60 Wt% matrix agent. In claim 1, the formulation contains between about 45 Wt% and about 55 Wt% matrix agent, or about 48 Wt% and about 52 Wt% matrix agent.

In other disclosed embodiments, the formulation contains between about 35 Wt% and 55 Wt% matrix agent. Particularly, the formulation contains between about 40 Wt% and about 50 Wt% matrix agent, or about 43 Wt% and about 47 Wt% matrix agent.

In other disclosed embodiments, the formulation contains between about 30 Wt% and 50 Wt% matrix agent. Particularly, the formulation contains between about 35 Wt% and about 45 Wt% matrix agent, or about 38 Wt% and about 42 Wt% matrix agent.

In other disclosed embodiments, the formulation contains between about 25 Wt% and 45 Wt% matrix agent. Particularly, the formulation contains between about 30 Wt% and about 40 Wt% matrix agent, or about 33 Wt% and about 37 Wt% matrix agent.

In other disclosed embodiments, the formulation contains between about 20 Wt% and 40 Wt% matrix agent. Particularly, the formulation contains between about 25 Wt% and about 35 Wt% matrix agent, or about 28 Wt% and about 32 Wt% matrix agent.

In other disclosed embodiments, the formulation contains between about 15 Wt% and 35 Wt% matrix agent. Particularly, the formulation contains between about 20 Wt% and about 30 Wt% matrix agent, or about 23 Wt% and about 27 Wt% matrix agent.

The dosage form of the present disclosure can also be rendered abuse deterrent by incorporating at least one plasticizer in the formulation. The plasticizer may provide the dosage form with a waxiness upon exposure to physical or mechanical forces, such as pulverizing or grinding. Suitable plasticizers may be selected from the group consisting of polyalkalene glycols (e.g., polyethylene glycol and polyethylene glycol monomethyl ether), acetyltributyl citrate, acetyltriethyl citrate, castor oil, diacetylated monoglycerides, dibutyl sebacate, diethyl phthalate, glycerin, propylene glycol, pullulan, sorbitol sorbitan solution, triacetin, tributyl citrate and triethyl citrate. In one embodiment, the plasticizer is polyethylene glycol.

In claim 1, the plasticizer comprises a polyalkalene glycol.

The performance of the plasticizer is dependent on the size and the amount of plasticizer present in the formulation. In one embodiment, the plasticizer cannot be filtered and/or separated from a resulting solution of water and/or alcohol. The formulation of the present disclosure may include a plasticizer having a molecular weight between about 1K Daltons and about 15K Daltons. Particularly, the molecular weight is between about 2K Daltons and about 14K, about 3K and about 13K Daltons, about 5K and about 10K Daltons, or about 7K and about 9K Daltons.

The formulation, or final dosage form, may contain between about 5.0 Wt% and about 60 Wt% plasticizer. In one embodiment, the formulation contains between about 20 Wt% and about 60 Wt% plasticizer. Particularly, the formulation contains between about 30 Wt% and about 50 Wt% plasticizer, or about 35 Wt% and about 45 Wt% plasticizer. In another embodiment, the formulation contains between about 15 Wt% and about 30 Wt% plasticizer. Particularly, the formulation contains between about 20 Wt% and about 28 Wt% plasticizer, or about 23 Wt% and about 27 Wt% plasticizer. In another embodiment, the formulation contains between about 5.0 Wt% and about 20 Wt% plasticizer. Particularly, the formulation contains between about 8.0 Wt% and about 17 Wt% plasticizer, or about 10 Wt% and about 15 Wt% plasticizer.

The dosage form of the present disclosure may also contain a filler/binder excipient (herein "filler"). A filler may be added to provide or increase the consistency of the extrudate for processing into a final dosage form. The filler may also help with hardness and dissolution of the dosage form. The filler may be a known excipient for use in pharmaceutical formulations that, upon extrusion, is capable of producing an extrudate that holds its shape. In some embodiments, the filler may have a melting temperature above the extrusion process temperatures, such as a melting temperature above about 80 °C, 90 °C, 100 °C, 110 °C, 120 °C, or 130 °C. The filler may also be a material that exhibits excellent flow and compression properties, wherein such flow and compression properties are measured by traditional methods known to persons skilled in the art of pharmaceutical formulations.

The filler may be selected from the group consisting of microcrystalline cellulose, silicified microcrystalline cellulose, powdered cellulose, maize starch, modified maize starch, potato starch, lactose monohydrate, lactose anhydrous, mannitol, dibasic calcium phosphate dihydrate, anhydrous dibasic calcium phosphate, magnesium carbonate, erythritol, trehalose and mixtures thereof. In one embodiment, the filler is microcrystalline cellulose and/or lactose monohydrate.

In some embodiments, the filler may also be useful as a disintegrant. For example, the inclusion of 10% or more of a filler, e.g., microcrystalline cellulose, also acts as a disintegrant.

The performance of the filler and the formulation is also dependent on the amount of filler present in the formulation. The formulation, or final dosage form, may contain between about 0 Wt% and about 40 Wt% filler. In one embodiment, the formulation contains between about 10 Wt% and about 40 Wt% filler. Particularly, the formulation contains between about 20 Wt% and about 40 Wt% filler, about 30 Wt% and about 40 Wt% filler, about 32 Wt% and about 38 Wt% filler, or about 34 Wt% and about 36 Wt% filler. In another embodiment, the formulation contains between about 25 Wt% and about 35 Wt% filler. Particularly, the formulation contains between about 27 Wt% and about 33 Wt% filler, or about 29 Wt% and about 31 Wt% filler. In another embodiment, the formulation contains between about 20 Wt% and about 30 Wt% filler. Particularly, the formulation contains between about 22 Wt% and about 28 Wt% filler, or about 24 Wt% and about 26 Wt% filler. In another embodiment, the formulation contains between about 10 Wt% and about 20 Wt% filler. Particularly, the formulation contains between about 12 Wt% and about 18 Wt% filler, or about 14 Wt% and about 16 Wt% filler. In another embodiment, the formulation of the present disclosure excludes a filler.

In one embodiment, the formulation includes a disintegrant. A disintegrant promotes disintegration of the pill, and dissolution of the active substance, after administration and upon contact with water. The disintegrant may be selected from sodium starch glycolate, cross-linked polyvinylpyrrolidone, sodium bicarbonate/citric acid, alginic acid or combinations thereof. The formulation, or final dosage form, may contain between about 1.0 Wt% and about 20 Wt% of disintegrant. Particularly, the formulation contains between about 1.0 Wt% and about 10 Wt% disintegrant. In another embodiment, the formulation of the present disclosure excludes a disintegrant.

In another embodiment, the formulation includes a dye. A dye is useful to distinguish or identify the pill of the present disclosure from other drug products. The dye may be selected from known dyes suitable for use in pharmaceutical formulations or approved by the FDA for such use. For example, the dye may be FD&C Blue No. 2 or a 50/50 Wt% blend of FD&C Blue No. 2 in polyethylene glycol. In one embodiment, the dye and polyethylene glycol blend cannot be substantially filtered and/or separated from a resulting solution of water and/or alcohol. The formulation, or final dosage form, may contain between about 0.10 Wt% and about 15 Wt% dye. Particularly, the formulation may contain between about 0.20 Wt% and about 12.5 Wt% dye, or about 0.50 Wt% and about 10 Wt% dye. In some embodiments, the formulation contains at least 0.1%, 0.2%, 0.3%, 0.5%, 0.8%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10%, 11%, 12%, 13%, 14%, 15%, and 20% dye. These values can also be used to define a range of dye present in the formulations, e.g., about 3.0% to about 5.0%. In another embodiment, the formulation of the present disclosure excludes a dye.

In another embodiment, the formulation includes a preservative or antioxidant. The preservative or antioxidant reduces or limits the degradation or deterioration of the abuse deterrent dosage form. For example, the components of the oral drug delivery system (e.g., active substances, matrix agents) may undergo degradation (e.g., oxidative reduction, chain cleavage) due to oxidation. Preventing degradation is essential to maintaining a proper release profile. For instance, the molecular weight of polyethylene oxide in the formulation affects the release profile of the active substance. The addition of a preservative or antioxidant in the formulation that reduces or eliminates the degradation of the molecular weight of polyethylene oxide is useful to maintain the release profile of the active substance.

The preservative or antioxidant may be selected from preservatives or antioxidants known to one skilled in the art for use in pharmaceutical formulations, such as citric acid, ascorbic acid, ascorbyl palmitate, butylated hydroxyanisole (BHA), butylated hydroxytoluene (BHT), erythorbic acid, hypophosphorous acid, lactobionic acid, monothioglycerol, potassium metabisulfite, propyl gallate, racemethionine, sodium bisulfite, sodium formaldehyde sulfoxylate, sodium metabisulfite, sodium sulfite, sodium thiosulfate, stannous chloride, sulfur dioxide and tocopherols. The formulation, or final dosage form, may contain between about 0.1 Wt% and about 2.0 Wt%, or about 0.25 Wt% and about 0.75 Wt% of preservative or antioxidant. In another embodiment, the formulation of the present disclosure excludes a preservative or antioxidant.

In some embodiments, the dosage form includes one or more agents that decrease the purity of the active substance in an alcohol solution or extraction product. The alcohol purity decreasing agent can reduce or limit the potential for abuse by decreasing the total weight percent of active substance found in the alcohol solution or alcohol based extraction by increasing the amount of other substances also found in the solution or extraction. For example, a common form of abuse involves extraction of active substance using ethanol, or ethanol solutions (e.g., 1% - 99% ethanol), resulting in a very pure powder. When introduced to an alcohol solution, components of the oral drug delivery system containing an alcohol purity decreasing agent (e.g., active substances, excipients) may become dissolved in the solution, creating a homogenous liquid which prevents extraction and subsequent alcohol evaporation to a very pure active substance. In one embodiment, the alcohol purity decreasing agent substantially decreases the purity of a resulting alcohol and/or water solution or powder. In another embodiment, the alcohol purity decreasing agent is not soluble in water. The dosage form may contain between about 0.1 wt% to 40 wt% alcohol purity decreasing agent. In some embodiments, the formulation contains at least 0.1%, 0.25%, 0.5%, 1.0%, 2.0%, 3.0%, 4.0%, 5.0%, 6.0%, 7.0%, 8.0%, 9.0%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40% alcohol purity decreasing agent. These values can also be used to define a range of agent present in the formulations, e.g., about 5.0% to about 15.0%. In another embodiment, the dosage form of the present disclosure excludes an alcohol purity decreasing agent.

The alcohol purity decreasing agent may be selected from excipients known to one skilled in the art for use in pharmaceutical formulations, such as alginic acid, calcium acetate, carbomers, carboxymethylcellulose, ethylcellulose, gelatin, hydroxyethylcellulose, hydroxypropyl cellulose, methylcellulose, poloxamers, polyvinyl alcohol, polyvinyl acetate, polyvinylpyrrolidone, and sodium alginate. In a specific embodiment, the agent is calcium acetate.

In some embodiments, the alcohol purity decreasing agent reducing the purity of a resulting alcohol and/or water solution or powder by at least 10%, 11%, 12%, 13%, 14%, 15%, 16%, 17%, 18%, 19%, 20%, 25%, 30%, 35%, 40%, 45%, 50%, 60%, 70%, 80%, 90% or 95%. These values can also be used to define a range of purity reduction, e.g., about 10% to about 30%.

The formulation may additionally include at least one additive independently selected from surfactants, bulking agents, lubricants, flavorings or combination thereof.

The abuse deterrent pill of the present disclosure is capable of immediate release of the active substance. The dosage form may be manufactured to provide a composition exhibiting an immediate release profile of at least one active substance. As used herein, "immediate release" refers to a dosage form that releases the active substance or a pharmaceutically acceptable salt thereof, substantially completely into the gastrointestinal tract of the user within a period of less than an hour, and often less than about 45 minutes from ingestion. In one embodiment, the amount of active substance released from the dosage form, e.g. oxycodone HCl, by exposure to a deairated water within 45 minutes is greater than or equal to 75%. In another embodiment, the amount of active substance released from the dosage form, e.g. hydrocodone bitartrate/acetaminophen, by exposure to a 0.1 N hydrochloric acid solution within 30 minutes is great than or equal to 90%.

In one embodiment, the formulation of the present disclosure releases greater than or equal to about 75% of the active substance within 45 minutes after administration or via dissolution testing. Particularly, the formulation releases greater than or equal to about 80%, about 85%, about 90%, or about 95% of the active substance within 45 minutes after administration or via dissolution testing.

In other embodiments, the formulation of the present disclosure releases greater than or equal to about 90% of the active substance within 30 minutes after administration or via dissolution testing. Particularly, the formulation releases greater than or equal to about 92%, about 94%, about 96%, or about 98% of the active substance within 30 minutes after administration or via dissolution testing.

The formulation of the present disclosure is abuse deterrent and does not rapidly release the active substance within a relatively short time after administration or dissolution testing begins. In some embodiments, the formulation of the present disclosure releases less than about 95% of the active substance within 20 minutes after administration or via dissolution testing. Particularly, the formulation releases less than about 90% of the active substance, less than about 85%, less than about 80%, less than about 75%, less than about 70%, less than about 65%, or less than about 60% within 20 minutes after administration or via dissolution testing.

In other embodiments, the formulation of the present disclosure releases less than about 95% of the active substance within 15 minutes after administration or via dissolution testing. Particularly, the formulation releases less than about 90% of the active substance, less than about 85%, less than about 80%, less than about 75%, less than about 70%, less than about 65%, or less than about 60% within 15 minutes after administration or via dissolution testing.

In one embodiment, the present disclosure relates to an oral, immediate release, abuse deterrent dosage form comprising an active substance susceptible to abuse, wherein less than about 95% of the active substance is released from the dosage form within 20 minutes following administration, and wherein greater than or equal to 75% of the active substance is released from the dosage form within 45 minutes following administration. In another embodiment, the present disclosure relates to an oral, immediate release, abuse deterrent dosage form comprising an active substance susceptible to abuse, wherein less than about 95% of the active substance is released from the dosage form within 15 minutes following administration, and wherein greater than or equal to 90% of the active substance is released from the dosage form within 30 minutes following administration.

The formulation, or abuse deterrent pill, may also include at least one physical barrier to reduce abuse. The physical barrier may be the inability of the pill to be abused by pulverizing and swallowing, pulverizing and snorting, pulverizing and injecting, or combinations thereof. For example, the abuse deterrent pill of the present disclosure may be incapable of being significantly pulverizing by physical or mechanical force.

One of the most common means of abuse of an orally administered opioid analgesic involves the manipulation of the oral dosage form in order to cause rapid delivery to the bloodstream via nasal insufflation. In order for insufflation to be used as an effective means of abuse, the original dosage form must be manipulated so as to decrease the particle size of the ingested drug to about 0.5 mm or less. A particle size of about 0.5 mm or less is necessary for effective intranasal absorption to occur. By limiting the quantity of particles under about 0.5 mm that an abuser can obtain by reasonable methods, one can render insufflation ineffective as a means of abuse. One way this physical barrier may be created is by capturing the active substance susceptible to abuse in a plastic matrix which is resistant to being physically broken down to produce particles smaller than about 0.5 mm.

The dosage form of the present disclosure can inhibit manipulation by grinding or pulverizing using common equipment, such as a coffee grinder. For example, the formulation deters abuse by limiting the particle size to which the formulation may be ground. The formulation prevents the pill, or at least substantial portions of the pill, from being ground in particles having a particle size of about 0.5 mm or less that may pass through the membrane of the nasal cavity. The dosage form can also significantly limit the extraction of the active substance by common solvents (e.g., cold water or distilled aqueous ethanol) from the formulation. For example, the formulation deters abuse by limiting the ability of persons to extract the active substance from the formulation (either intentionally or unintentionally), such that the active substance cannot easily be concentrated for parenteral administration. The abuse deterrent formulation may also include, but does not require, the incorporation of other deterrents such as antagonists or irritants.

In one embodiment, the abuse deterrent pill of the present disclosure may be incapable of being crushed by grinding into a form that may be abused. In a coffee grinder assay as described in Example 1 (e.g., grinding in a coffee grinder at about 20,000+ rpm and for about 30-60 seconds) the pill remains in a form that may not be abused. The coffee grinder assay may be performed using a commercial coffee grinder, or equivalent, capable of grinding abuse deterrent pills. The pills tested using the coffee grinder assay have a substantial portion of the resulting particles with a particle size which is not able to be abused, i.e. intranasal administered. Abuse deterrent pills having a substantial amount of such particles reduce the incentive or cost-effectiveness of persons to abuse the formulations. For example, a potential abuser who can only access for intranasal administration less than about 50% of the active substance will be deterred from abusing the formulation.

Upon exposure to a grinding force (e.g., the coffee grinder assay or equivalent), the abuse deterrent pill may be grinded into particles wherein at least about 50 Wt% of the grinded particles have a particle size greater than about 0.5 mm. Particularly, upon exposure to a grinding force, the abuse deterrent pill may be grinded into particles wherein at least about 55 Wt% of the grinded particles, 60 Wt% of the grinded particles, 65 Wt% of the grinded particles, 70 Wt% of the grinded particles, 75 Wt% of the grinded particles, 80 Wt% of the grinded particles, 85 Wt% of the grinded particles, 90 Wt% of the grinded particles, or 95 Wt% of the grinded particles have a particle size greater than about 0.5 mm.

In another embodiment, the abuse deterrent pill of the present disclosure may be capable of forming a hydrogel upon exposure to an aqueous or semi-aqueous solution. The formation of the hydrogel deters abuse by limiting the ability of persons to extract the active substance from the formulation, such that the active substance cannot easily be concentrated for parenteral administration.

In some embodiments, the abuse deterrent pill of the present disclosure is capable of releasing its dye upon introduction to an aqueous or semi-aqueous solution. The dye provides a visual deterrent to abuse via parenteral administration by giving the solution a turbid and/or deep color. In some embodiments, the dye is not capable of being separated from the solution via nylon, PTFE, coffee or other readily available filters or filtering techniques.

In another embodiment, the present disclosure relates to a process for the production of an oral, immediate release, abuse deterrent pill containing at least one active substance susceptible to abuse comprising processing a uniform blend of the at least one active substance susceptible to abuse, a matrix agent and a plasticizer by hot melt extrusion to produce an extrudate. The extrudate may therein be formed using a forming unit into the pill.

Hot melt extrusion is a processing technique used to make the formulations and compositions of the present disclosure because it allows for the creation of homogeneous polymer matrices with specific abuse deterrent properties. For example, by varying the formulation and the processing parameters specific properties such as dissolution time, pulverization resistance, material processability, and stability can be selectively modified. Formulations that include polymer matrix agents (e.g., polyethylene oxide) can provide a unique advantage as they allow for formulations in which release characteristics can be controlled while also creating a physical barrier that prevents abuse (e.g., through means of nasal inhalation or intravenous injection). Furthermore, in a hot melt extrusion process, process analytic data can be provided in real time. The process may also be adapted for continuous process manufacturing procedure as opposed to traditional batch to batch processing.

The abuse deterrent pill of the present disclosure may be formed by hot melt extrusion using commercially available extruders, such as a twin screw extruder. Several factors of the extrusion process may affect the final extrudate including: screw design (sheer rating), screw speed, temperature profile, feed rate, dwell time, die pressure and die size. These factors may be varied to obtain an extrudate with desired processing capabilities such that the extrudate is uniform, holds its shape, and is capable of being formed into pills by a forming unit.

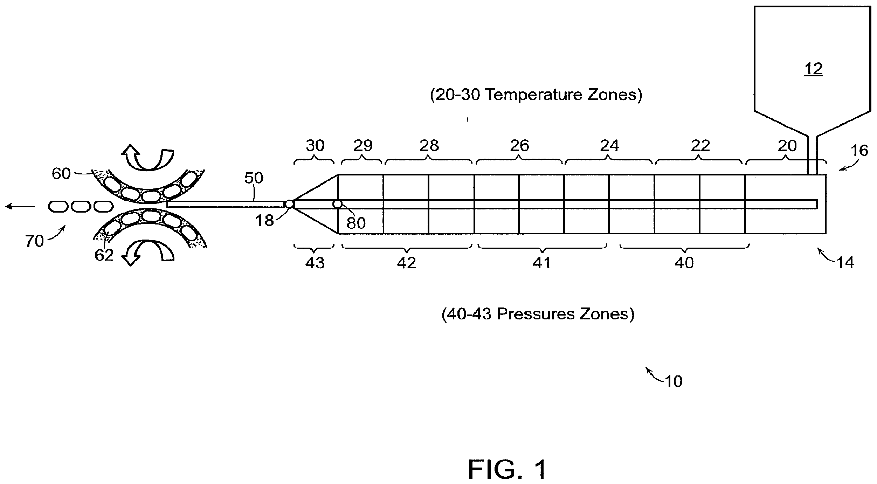

An exemplary extruder and forming unit system (10) is shown in Figure 1. The extruder (14) includes a hopper or feeding unit (12) wherein a uniform blend of the formulation is made or transferred to. The uniform blend is fed into the inlet (16) of the extruder (14) by starve feeding via a gravimetric or volumetric dosing unit. The formulation of the present disclosure is preferably uniformly blended prior to introduction to the extrusion process. Insufficient blending of the components may produce a non-uniform extrudate and non-uniform abuse deterrent pills having inconsistent amounts of active substance. Over-blending may produce a poorly performing formulation. The blending process may be monitored using a process analytical technique to determine when a uniform blend is achieved. In one embodiment, the mixing bin or hopper (12) may be equipped with a near-infrared (NIR) monitoring system for in-line, continuous monitoring of the blend.

In one embodiment, monitoring of the blending process by NIR involves preparing a NIR standard spectrum for each formulation. The NIR standard spectra may be prepared empirically by monitoring the blending of different batches of the formulation. The blending conditions and/or extrusion process may be correlated with NIR spectra to determine a NIR standard spectrum for a given dosage form. Once the optimum NIR monitoring spectrum and conditions are determined, the formulation is blended until the NIR standard is achieved. One of ordinary skill in the art armed with the present disclosure can implement a near-infrared monitoring system for in-line, continuous monitoring of the blend.

The extruder (14) then processes the blend into a melt and passes the extrudate (50) out of the extruder (14) through a die section (30) and through a die outlet (18). The extruder (14) may have temperature zones (20-30) and pressure zone (40-43). These zones may include components to heat and pressurize the extruder (14) or may include sensors to measure the temperature and/or pressure of each particular zone.

As used herein the term melt temperature refers to the temperature at which an excipient changes from solid to liquid state. As used herein the term softening temperature refers to the temperature at which an excipient changes from solid form into a malleable, dynamic solid.

The temperature profile of the extruder (14) is important to obtain a uniform extrudate (50) with little to no degradation products. Heat may be applied to soften, and in some embodiments to melt, the excipients (e.g., matrix agent, plasticizer) to form a homogenous matrix to encapsulate the active substance. The extruder temperature profile, or the temperatures in the extruder zones (20-30), is preferably kept below the melting point, and often the degradation point, of the active substance.

For example, the melting temperature of polyethylene oxide is about 67 °C and of polyethylene glycol is about 63 °C. Common active substances begin to melt at temperatures much higher than this. For example, the melt temperature of oxycodone HCl is about 219 °C and of hydrocodone bitartrate is about 147 °C. Preferably, the temperature of one or more of the zones (20-30) is kept at or below the melting point of the active pharmaceutical ingredients. In particular, the temperature of one or more of the zones (20-30) is kept below about 120 °C, 110 °C, 100 °C, 90 °C, 80 °C, 75 °C, 70 °C, 65 °C, or 60 °C.

In one embodiment, the temperature of at least one of the extruder zones (20-30) is kept at or below the melting point of the active pharmaceutical ingredients. Particularly, the temperature of at least one of the zones is kept below about 120 °C, about 110 °C, about 100 °C, about 90 °C, about 80 °C, about 75 °C, about 70 °C, about 65 °C, or about 60 °C.

In another embodiment, the temperature of at least two of the extruder zones (20-30) is kept at or below the melting point of the active pharmaceutical ingredients. Particularly, the temperature of at least two of the zones is kept below about 120 °C, about 110 °C, about 100 °C, about 90 °C, about 80 °C, about 75 °C, about 70 °C, about 65 °C, or about 60 °C.

In another embodiment, the temperature of at least three of the extruder zones (20-30) is kept at or below the melting point of the active pharmaceutical ingredients. Particularly, the temperature of at least three of the zones is kept below about 120 °C, about 110 °C, about 100 °C, about 90 °C, about 80 °C, about 75 °C, about 70 °C, about 65 °C, or about 60 °C.

In another embodiment, the temperature of at least four of the extruder zones (20-30) is kept at or below the melting point of the active pharmaceutical ingredients. Particularly, the temperature of at least four of the zones is kept below about 120 °C, about 110 °C, about 100 °C, about 90 °C, about 80 °C, about 75 °C, about 70 °C, about 65 °C, or about 60 °C.

In another embodiment, the temperature of at least five of the extruder zones (20-30) is kept at or below the melting point of the active pharmaceutical ingredients. Particularly, the temperature of at least five of the zones is kept below about 120 °C, about 110 °C, about 100 °C, about 90 °C, about 80 °C, about 75 °C, about 70 °C, about 65 °C, or about 60 °C.

In another embodiment, the temperature of at least six of the extruder zones (20-30) is kept at or below the melting point of the active pharmaceutical ingredients. Particularly, the temperature of at least six of the zones is kept below about 120 °C, about 110 °C, about 100 °C, about 90 °C, about 80 °C, about 75 °C, about 70 °C, about 65 °C, or about 60 °C.

In another embodiment, the temperature of all of the extruder zones (20-30) is kept at or below the melting point of the active pharmaceutical ingredients, with the optional exception of the die zone. Particularly, the temperature of all of the zones is kept below about 120 °C, about 110 °C, about 100 °C, about 90 °C, about 80 °C, about 75 °C, about 70 °C, about 65 °C, or about 60 °C, with the optional exception of the die zone.

The temperature of the die (18, 30) may be maintained at a slightly higher temperature than the temperature of one or more of the other zones. In some embodiments, the die temperature (18, 30) is held at or slightly above the melting point of the extrudate, or the matrix and plasticizer, to ensure a uniform extrudate (50) exiting the die outlet (18).

The extruder (14) also has a pressure profile. Pressure is important to melt the excipients to make mixing more efficient and to force the extrudate (50) through the die outlet (18) to exit the extruder (14) in a consistent manner. Particularly, the pressures in the zones and also the pressure at the die outlet (18), is kept at or above about 5 bar, about 10 bar, about 15 bar, about 20 bar, about 30 bar, about 40 bar, about 50 bar, about 60 bar, about 70 bar, about 80 bar, about 90 bar, about 100 bar or about 110 bar.

In one embodiment, the pressure of one or more of the pressure zones (40-43) in the extruder (14) is kept at a high enough pressure to achieve melting, compression, and mixing of the matrix and plasticizing agents with non-melting excipients (e.g., API, filler, disintegrants, and antioxidant) while the temperature of one or more of the temperature zones (20-30) is at or slightly below the melting point at standard pressure of these agents. The increased pressure allows for more efficient mixing due to compaction and shearing forces without having to dramatically increase temperature. These lower temperatures reduce, or substantially eliminate, the formation of degradation products from the active substances. In one embodiment, the pressure produced on the die (43) of the extruder (14) is kept sufficiently high enough to reduce pulsating flow and ensure a uniform extrudate (50) is delivered though the die outlet (18). A sufficiently high pressure assists in compacting the homogenous melt into a processable strand of desired diameter.

In one embodiment, the pressure of at least one of the pressure zones (40-43) is kept at a high enough pressure to achieve melting, compression, and mixing of the matrix and plasticizing agents with the active substance and any non-melting excipients. Particularly, the pressure of at least one of the zones is kept at or above about 5 bar, about 10 bar, about 15 bar, about 20 bar, about 30 bar, about 40 bar, about 50 bar, about 60 bar, about 70 bar, about 80 bar, about 90 bar, about 100 bar or about 110 bar

In another embodiment, the pressure of at least two of the pressure zones (40-43) is kept at a high enough pressure to achieve melting, compression, and mixing of the matrix and plasticizing agents with the active substance and any non-melting excipients. Particularly, the pressure of at least two of the zones is kept at or above about 5 bar, about 10 bar, about 15 bar, about 20 bar, about 30 bar, about 40 bar, about 50 bar, about 60 bar, about 70 bar, about 80 bar, about 90 bar, about 100 bar or about 110 bar

In another embodiment, the pressure of at least three of the pressure zones (40-43) is kept at a high enough pressure to achieve melting, compression, and mixing of the matrix and plasticizing agents with the active substance and any non-melting excipients. Particularly, the pressure of at least three of the zones is kept at or above about 5 bar, about 10 bar, about 15 bar, about 20 bar, about 30 bar, about 40 bar, about 50 bar, about 60 bar, about 70 bar, about 80 bar, about 90 bar, about 100 bar or about 110 bar

In another embodiment, the pressure of all of the pressure zones (40-43) is kept at a high enough pressure to achieve melting, compression, and mixing of the matrix and plasticizing agents with the active substance and any non-melting excipients. Particularly, the pressure of all of the zones is kept at or above about 5 bar, about 10 bar, about 15 bar, about 20 bar, about 30 bar, about 40 bar, about 50 bar, about 60 bar, about 70 bar, about 80 bar, about 90 bar, about 100 bar or about 110 bar

The melt extrudate may be optionally analyzed within the extruder (14) using near-infrared technology. NIR spectroscopy can be used as a non-invasive alternative to high performance liquid chromatography techniques. A NIR probe (80) may be included within the extruder (14). The wavelengths and intensities at which raw organic materials of the melt extrudate absorb light energy can be plotted to produce spectra to compare against a standard. With the spectrum of the API known, it can be used to determine and monitor the % wt of the active pharmaceutical ingredient present in the extrudate in real time.

The extrudate from an extruder is directly formed into a pill using a forming unit, provided that the size or shape of the extrudate may be adjusted prior to introduction to the forming unit (e.g., via a rope sizer). In some embodiments, the extrudate is directly formed into a dosage form without a further processing step, such as a cutting or milling step. The forming unit may be a unit capable of forming the pill without cutting or milling the extrudate. The forming unit may be a calendar, rotary, or a chain forming machine. As shown in Figure 1, the extrudate (50) may be shaped into the abuse deterrent form (70) by a forming unit (60). In one embodiment, the extrudate (50) is shaped into the abuse deterrent form (70) by a calendaring process.

The forming unit (60) may comprise two rotating components each having molds (62) inset in the rotating components and aligned such that the molds (62) overlap with each other as the rotating components interface. When the extrudate (50) is guided between the rotating components of the forming unit (60), the offset and aligned molds (62) (or cavities) accept the extrudate and form the extrudate into the dosage form as provided by the shape of the molds (62), provided a sufficient amount of extrudate is guided between and supplied to the rotating components.

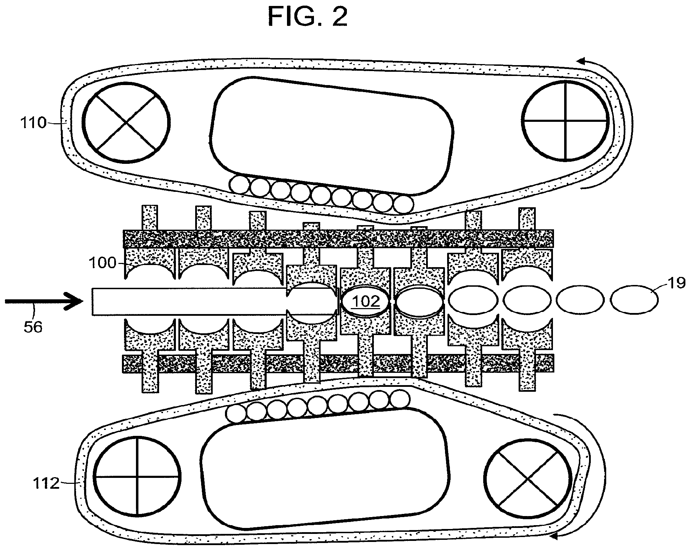

In another embodiment, the forming unit may also comprise of a rotating set of punch dies with accompanying pinching ring, e.g. a chain die forming unit. Figure 2 shows an embodiment of a chain forming unit. The chain forming unit includes an upper and lower chain system (110 and 112) and tooling (100) to form an incoming extrudate (56) into formed pills (19). When the extrudate (56) is fed into the chain die forming unit, the ring tooling (100) pinches the extrudate (56) to the exact weight of the finished pill and simultaneously presses it into a final form by the punches via a cam track. In one embodiment, the centripetal forces produced by the rotation of the machine aid in the ejection of the final pill form (19).

The extruder / forming unit system (10) may also be equipped with an additional component or transfer unit to assist the transfer of the extrudate (50) from the extruder (14) to the forming unit (60). The transfer unit may be capable of controlling the temperature, pressure, environment and/or shape of the extrudate. For example, the transfer unit may include heated/cooled sizing rollers which process the extrudate (50) into a consistent size (e.g., diameter) before entering the forming unit, cooling air jets, and extrudate diameter monitoring. The transfer unit may also be capable of guiding the extrudate into and between the rotating components of the forming unit (60).

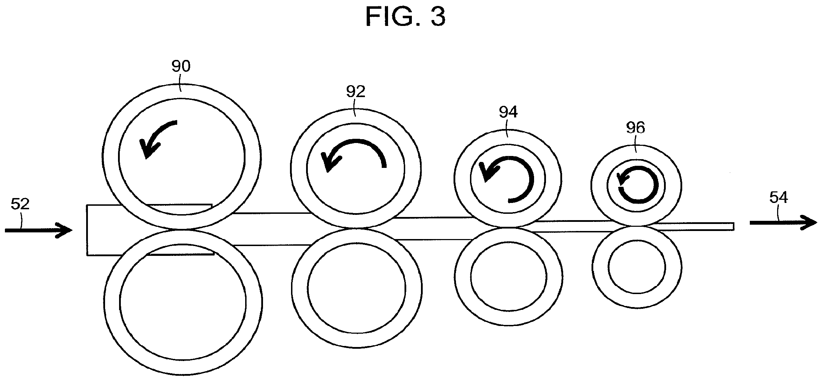

For example, the extrudate may be adjusted by an apparatus that re-sizes the extrudate, re-shapes the extrudate, or both. Figure 3 shows an embodiment of an extrudate sizing apparatus (e.g., rope sizer). The rope sizer includes a number of consecutive rollers (90-96) to re-size or re-shape an incoming extrudate (52), either from the extruder (14) or from another step. The number, shape and orientation of the rollers (90-96) may vary depending on the degree of re-sizing and/or re-shaping desired. In some embodiments, the extrudate will be re-sized into a smaller diameter extrudate. In these embodiments, the rotating rollers will rotate at consecutively faster speeds. As such, the re-sized and/or re-shaped extrudate having a smaller diameter will be moving at a faster speed exiting the rope sizer.

The size and shape of the extrudate (50) may be designed to efficiently interact with different shaped molds (62). For example, an oval shaped extrudate may be formed to interact with a wide and shallow set of molds (62). Also, the speed and mass (or volume) of the extrudate (50) may be designed to efficiently interact with the size and speed of the forming unit. The speed and mass (or volume) of the extrudate (50) guided between the rotating components of the forming unit (60) should be sufficient to fill each set of molds completely with no voids.

The size and shape, and the speed and mass (or volume) of the extrudate (50) as well as size and shape of the molds (62) and the speed of the forming unit may be matched to reduce the amount of excess extrudate that is not formed into the dosage form (e.g., reduce waste). The two processes may be synchronized by attaching both to the same drive system. Preferably, the forming unit is capable of forming abuse deterrent pills from the extrudate wherein more than about 90% of the extrudate is utilized (e.g., formed into the dosage form). More preferably, the forming unit utilizes more than about 95% of the extrudate. Even more preferably, the forming unit utilizes more than about 99% of the extrudate.

The molds (62) may optionally be formed with a non-uniform bottom or lower surface to allow for easy removal of the pill after formation. The molds (62) may also have markings in the bottom or lower surface to provide marking on the abuse deterrent pills upon formation.

After formation, the quality, volume and weight of each pill may be determined using an automated optical inspection technique. The optional inspection technique combines a weight determination step and a visual inspection step into a single step. For example, the visualization step may include taking multiple pictures of each pill. From these pictures, an estimated volume is determined. The estimated volume and the pre-determined density of the composition of the formulation may provide an estimated weight for each pill. Those pills that satisfy certain quality, volume and weight criteria will pass the optical inspection.

In another embodiment, the present disclosure relates to an process for the production of an oral, immediate release, abuse deterrent pill containing at least one active substance susceptible to abuse comprising combining the at least one active substance susceptible to abuse, a matrix agent, and a plasticizer in a hopper to form a mixture; blending the mixture in the hopper until a uniform blend is achieved; monitoring the mixture during blending using a process analytical technique to determine when a uniform blend is achieved; feeding the uniform blend into an extruder; processing the uniform blend by hot melt extrusion to produce an extrudate; optionally monitoring of the extrudate at the die head via PAT NIR probe; transferring the extrudate to a forming unit using a transfer line capable of controlling the temperature, pressure, environment, and/or shape of the extrudate; forming the extrudate using the forming unit into the pill; and determining the quality, volume and weight of the pill using an optical inspection technique.

In another embodiment, the present disclosure relates to a method of treating pain comprising administering to an individual in need thereof a therapeutically effective amount of a dosage form as described herein. The dosage form provides rapid onset of analgesia for the treatment of moderate to severe pain.

When an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

The present invention is further defined in the following Examples. It should be understood that these Examples, while indicating preferred embodiments of the invention, are given by way of illustration only.

### Examples

The following describe embodiments within the scope of the claims, and reference examples. The latter are provided for the useful understanding of the invention. Dosage forms according to claim 1 comprise *i.a.* a matrix agent which is polyethylene oxide which has an average molecular weight between 50K Daltons and 150K Daltons, and which is 45wt% to 55 wt% of the dosage form.

### Example 1

Abuse deterrent formulations were prepared containing acetaminophen. Acetaminophen was utilized as a tracer in place of oxycodone HCl due to its availability, cost, similar particle size, and solubility profile. Tables 1-3 show the exemplary ranges of components for the abuse deterrent formulations.

**Table 1: Exemplary Abuse Deterrent Formulation Ranges**

| Components | Wt % |
|---|---|
| Active Substance | 0.1-30.0 |
| Matrix Agent (50K to 300K Daltons) | 10.0-90.0 |
| Plasticizer (1K to 15K Daltons) | 5.0-60.0 |
| Filler | 0.0-40.0 |
| Disintegrant | 0.0-10.0 |
| Antioxidant(s) | 0.0-2.0 |
| Dye | 0.0-20.0 |
| Alcohol Purity Decreasing Agent | 0.0-30.0 |

**Table 2: Exemplary Abuse Deterrent Formulation Ranges**

| Components | Wt % |
|---|---|
| Active Substance | 0.1-15.0 |
| Polyethylene Oxide (50K to 300K Daltons) | 10.0-90.0 |
| Polyethylene Glycol (1K to 15K Daltons) | 5.0-60.0 |
| Microcrystalline Cellulose | 20.0-40.0 |
| Crospovidone (Disintegrant) | 0.0-10.0 |
| Citric Acid (Antioxidant 1) | 0.0-1.0 |
| Butylated hydroxytoluene (Antioxidant 2) | 0.0-1.0 |
| Calcium Acetate | 0.0-10.0 |

**Table 3: Exemplary Abuse Deterrent Formulation Ranges**

| Components | Wt % |
|---|---|
| Active Substance | 5.0-10.0 |
| Polyethylene Oxide (50K to 300K Daltons) | 30.0-50.0 |
| Polyethylene Glycol (1K to 15K Daltons) | 15.0-30.0 |
| Lactose Monohydrate | 5.0-15.0 |
| Disintegrant | 2.5-7.5 |
| Antioxidants | 0.5-1.5 |
| Dye | 0.5-1.5 |
| Alcohol Purity Decreasing Agent | 0.0-30.0 |

Immediate release abuse deterrent pills containing acetaminophen were manufactured according to the formulation provided in Table 1.

The formulation was blended prior to extrusion. Extrusion was performed by means of a twin screw extruder of type Coperion ZSK26. To achieve a uniform extrudate with good processing capabilities a medium sheer screw design was used at a relatively slow screw speed (120RPM). The temperature profile was designed to immediately melt the matrix and plasticizer agents (e.g., the polyethylene oxide and polyethylene glycol). Thereafter, the temperature was adjusted to be at or slightly below the melting temperature of the extrudate at standard pressure to achieve mixing. Adequate mixing was achieved by maintaining high pressures in the extruder.

At times, the die was heated to a temperature above the general melting temperature of the extrudate. It was found that at die temperatures at the melting temperature of the extrudate, the portion of the extrudate in contact with the inside die surface sheared off due to friction. An increase in die temperature allowed the outside surface of the extrudate to melt and slide along the die producing a uniform extrudate. Operating temperatures and pressures are provided in Table 5. The temperature and pressure zones in Table 5 correspond to the zones shown in Figure 1.

**Table 4: Extrusion Temperature and Pressure**

| | Temp | Pressure |
|---|---|---|
| Zone 1 | 63-67 °C | |
| Zone 2 | 58-62 °C | |
| Zone 3 | 58-62 °C | |
| Zone 4 | 57-61 °C | |
| Zone 5 | 57-61 °C | |
| Zone 6 | 57-61 °C | |
| Zone 7 | 57-61 °C | |
| Zone 8 | 57-61 °C | |
| Zone 9 | 57-61 °C | |
| Zone 10 | 57-61 °C | |
| Die | 67-71 °C | |
| Melt Pressure | | 20-100 bar |

The temperature profile, feed rate, and die size all have an effect on the pressure produced on the die head. A die size of 3-5 mm was used. The temperature profile was kept relatively static. The feed rate was adjusted to maintain a consistent and high pressure on the die head of about 50 bar. A pressure maintained at the die head of about 50 bar or more produced uniform extrudates.

A Carver Press was then used to form the extrudate into pills. A Carver Press is a manual hand press which utilizes a free standing set of Natoli upper and lower punches that meet at the die. Dedicated tooling was made for the experiment in order to produce an embossed, 100 mg pill.

The extrudate was hand cut, based on weight (100 mg). The die was placed on top of the bottom punch, the cut extrudate was placed in the die cavity, and the top punch placed through the top section of the die. The cut extrudate was formed into a pill at no more than 1 metric ton of force using the Carver Press and Natoli die set.

## Dissolution Testing

The abuse deterrent pills prepared were tested for dissolution. Dissolution testing was performed with reference to USP Monograph on Oxycodone HCl Tablets. These tests were performed on a dissolution apparatus utilizing UPS <711> Apparatus II (Paddles), with 500 mL de-aerated water as media and a paddle speed of 50 rpm. Japanese Sinker Baskets (Part Number PSCUSBSK-JPMAG) were utilized. A 1 mL sample was pulled at 20 and 45 minutes and submitted for HPLC analysis. HPLC conditions were taken from the USP Monograph in order to observe the release of acetaminophen. The HPLC conditions are as follows: Injection Volume: 20 $\mu$L; Flow Rate 1.5 mL/min; Detection: UV at 295 nm; Column Temp: 25 °C; Autosampler Temperature: ambient; Gradient: Isocratic; and Runtime: 5 minutes. The specification for this dissolution testing was NLT 75% (Q=70%) at 45 minutes.

The weight percent of matrix agent (e.g., polyethylene oxide) in the formulation has a direct correlation with both the release profile and ADF properties. The effect of varying the weight percent of the matrix agent (e.g., polyethylene oxide) in the formulation on the release properties and ADF was tested. Two different molecular weight polyethylene oxides were used, namely 300K Daltons and 600K Daltons. The general formulation was tested for dissolution using the two different polyethylene oxides (i.e., 300K Daltons and 600K Daltons). For these experiments, polyethylene glycol was used to offset the differing weight percentages of PEO.

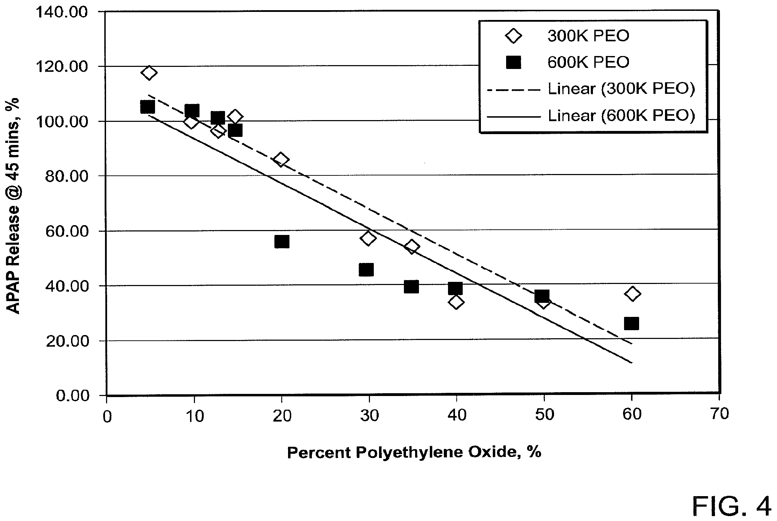

Figure 4 shows the percent release of the active substance (i.e., acetaminophen dissolution in 45 minutes) versus the weight percent of the matrix agent (e.g., polyethylene oxide or PEO) for two similar abuse deterrent pill formulations having different molecular weight matrix agents (e.g., 300K Daltons PEO vs. 600K Daltons PEO). Based on Figure 4, the more polyethylene oxide present in the formulation the lower the percent release of active after 45 minutes.

## Abuse Deterrent Testing - Coffee Grinder Assay

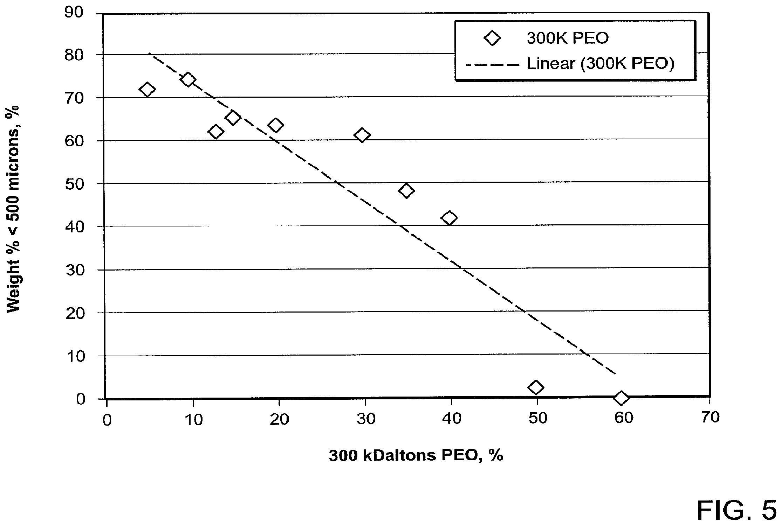

The abuse deterrent pills were also tested for resistance to pulverizing / grinding using a coffee grinder assay. The tested formulations contained only 300K Dalton polyethylene oxide. The Wt% of the polyethylene oxide was varied. Polyethylene glycol was again used to offset the differing weight percentages of PEO. Three (3) pills for each specific Wt% of polyethylene oxide were selected and placed in a commercially available coffee grinder (Mr. Coffee®, model number IDS55). The coffee grinder was run for 30 seconds with occasional pulsing. The grinded pills were tested for particle size analysis using a sonic sifter (screen size 35 Mesh) for 2 minutes. The 35 Mesh corresponds to a sieve size of 0.5 mm. The amount of particles below 0.5 mm for each formulation is shown in Figure 5. Figure 5 shows that with increasing weight percent of polyethylene oxide the ADF properties are enhanced. The percent of particles smaller than 0.5 mm decreases with increasing weight percent of polyethylene oxide. These results show that by increasing the content of polyethylene oxide the percentage of particles small enough to be abused through means of insufflation is decreased.

## Example 2

Immediate release abuse deterrent pills containing acetaminophen or oxycodone HCl were manufactured according to the formulation provided in Table 1.

A 150 g batch of each formulation was processed and formed into abuse deterrent pill by the process described in Example 1. Exemplary extruder operating temperatures and pressures are provided in Table 29.

**Table 5: Extrusion Temperature and Pressure**

|        | Temp      | Pressure |
|--------|-----------|----------|
| Zone 1 | 63-67 °C  |          |
| Zone 2 | 57-61 °C  |          |
| Zone 3 | 57-61 °C  |          |
| Zone 4 | 58-62 °C  |          |
| Zone 5 | 58-62 °C  |          |

**15**

(continued)

| | Temp | Pressure |
|---|---|---|
| Zone 6 | 58-62 °C | |
| Zone 7 | 58-62 °C | |
| Zone 8 | 58-62 °C | |
| Zone 9 | 58-62 °C | |
| Zone 10 | 58-62 °C | |
| Die | 68-72 °C | |
| Actual Melt Pressure | | 20-100 bar |

All of the abuse deterrent pills were tested for dissolution and abuse deterrence using the tests described in Example 1. All abuse deterrent pills exhibited a greater than 80% of particles having a particle size greater than 0.5 mm in the coffee grinder assay. All abuse deterrent pills also exhibited a more than 75% release within 45 minutes during dissolution testing.

In particular, abuse deterrent pills containing oxycodone HCl were tested for dissolution according to the test described in Example 1. The dissolution results, listed as percent label claim of oxycodone, are shown in Table 6.

**Table 6: Dissolution Data**

| Abuse Deterrent Pill, Test Formulation 1 | |
|---|---|
| Sample | % Label Claim |
| 20 Minutes (average of 3 pills) | 54.9 |
| 45 Minutes (average of 3 pills) | 81.3 |
| | |
| Abuse Deterrent Pill, Test Formulation 2 | |
| Sample | % Label Claim |
| 20 Minutes (average of 3 pills) | 57.8 |
| 45 Minutes (average of 3 pills) | 87.9 |

As shown in Table 6, the release profiles are comparable and both are consistent with immediate release formulations.

The abuse deterrent pills were put on accelerated stability under 40 °C / 75 %RH conditions for 1 and 2 Months. Dissolution testing was repeated. The results are shown in the Table 7.

**Table 7: Dissolution Data on Stability**

| Abuse Deterrent Pill, Test Formulation 1 | | |
|---|---|---|
| Sample | % Label Claim (1 Mo) | % Label Claim (2 Mo) |
| 20 Minutes (average of 6 pills) | 56.39 | 54.24 |
| 45 Minutes (average of 6 pills) | 85.18 | 81.63 |
| | | |
| Abuse Deterrent Pill, Test Formulation 2 | | |
| Sample | % Label Claim (1 Mo) | % Label Claim (2 Mo) |
| 20 Minutes (average of 6 pills) | 44.80 | 51.12 |
| 45 Minutes (average of 6 pills) | 75.75 | 80.24 |

As shown in Table 7, the release profile and the percent label claim for the abuse deterrent pills on stability are comparable to each other and to the original pills. The release profile for the abuse deterrent pills is consistent with immediate release formulations. The abuse deterrent pills exhibited excellent stability under accelerated conditions.

**Abuse Deterrent Testing - Coffee Grinder Assay**

The abuse deterrent pills containing oxycodone HCl were tested for resistance to pulverizing / grinding using the coffee grinder assay described in Example 1. Three (3) pills were selected and placed in the coffee grinder. The coffee grinder was run for 30 seconds with occasional pulsing. The grinded pills were tested for particle size analysis using a sonic sifter (screen size 35 Mesh). The results are shown in Table 8.

**Table 8: Pulverizing/Grinding Test (30 seconds)**

|  | Gross Wt (mg) | Tare Wt (mg) | Net Wt (mg) | % |
|---|---|---|---|---|
| 35 Mesh | 40461.70 | 40208.03 | 253.67 | 84 |
| Pan | 160822.45 | 160822.45 | 49.54 | 16 |
|  |  | Total | 303.21 | 100 |

The tested was repeated with the coffee grinder run for a longer period (2 minutes). The grinded pills were tested for particle size analysis using a sonic sifter(screen size 35 Mesh). The results are shown in Table 9.

**Table 9: Pulverizing/Grinding Test (2 minutes)**

|  | Gross Wt (mg) | Tare Wt (mg) | Net Wt (mg) | % |
|---|---|---|---|---|
| 35 Mesh | 40411.21 | 40209.15 | 202.06 | 73 |
| Pan | 160913.98 | 160839.25 | 74.73 | 27 |
|  |  | Total | 276.79 | 100 |

As shown in Tables 8 and 9, a majority of the grinded pills (84% and 73%) have a particle size larger than 0.5 mm. These pills are abuse deterrent compliant.

Additional abuse deterrent pills containing oxycodone HCl or hydrocodone bitartrate were tested for resistance to pulverizing / grinding using the coffee grinder test described above. The results are shown in Table 10. A majority of the grinded pills (89% +) has a particle size larger than 0.5 mm. These pills are abuse deterrent compliant.

**Table 10: Pulverizing/Grinding Test (2 minutes)**

|  | % particles > 500 microns | % particles < 500 microns |
|---|---|---|
| Test Formulation 3 (oxycodone HCl) | 89 | 11 |
| Test Formulation 4 (hydrocodone bitartrate) | 89 | 11 |

The abuse deterrent pills were also tested for hardness. Hardness testing was performed using a Sotax HT1 hardness testing machine. Two different formulations were tested for hardness, one containing acetaminophen and one containing oxycodone HCl.

For each formulation, three (3) formed pills were tested for hardness. All the hardness tested pills exhibited a hardness of > 999N.

**Example 3**

In order to attain the release profile required by USP standards for immediate release, the use of a disintegrant was tested. Several disintegrants were evaluated including croscarmellose sodium, sodium starch glycolate, cross-linked polyvinylpyrrolidone, sodium bicarbonate/citric acid and alginic acid. These disintegrants work though several methods such as swelling, wicking, and deformation in an aqueous environment to break up a formulation and thus increase surface area to aid in rapid API release.

Experiments utilizing polyethylene oxide 300K Daltons showed no significant improvement in dissolution rates with increased weight percent of croscarmellose sodium (CCS). CCS is water soluble and can form a gel at increasing percentages which may prevent disintegration. Sodium starch glycolate (SSG) was tested and found to rapid swell with minimal gelling effects. SSG was tested alone as well as with 5 Wt% and 10 Wt% sodium bicarbonate/citric acid acting as an effervescent. All combinations produced passing dissolution at 45 minutes. Additional experiments utilizing alginic acid produced similar passing dissolution results at 45 minutes.

Cross-linked polyvinylpyrrolidone was also tested. Cross-linked polyvinylpyrrolidone was tested alone as well as with 5 Wt% and 10 Wt% sodium bicarbonate/citric acid. Cross-linked polyvinylpyrrolidone also produced passing dissolution results. Cross-linked polyvinylpyrrolidone is highly hydrophilic and water insoluble. It acts through a wicking and swelling mechanism. Due to it being water insoluble, it does not form a gel in any concentration. Cross-linked polyvinylpyrrolidone provided better dissolution results regardless of Wt% sodium bicarbonate/citric acid which is believed to be due to its insoluble, non-gel forming nature. Thus, cross-linked polyvinylpyrrolidone was determined to be a preferred disintegrant for this formulation.

**Extraction Example**

The inclusion of one or more dyes in a drug formulation is one method to render a formulation abuse deterrent. Significant discoloration of an extraction product from a formulation subject to abuse can discourage a potential abuser from using (e.g., injecting or ingesting) the extraction product. A study was conducted to investigate the effect of dyes in the formulations of the present disclosure. Extraction products from whole or cut formulations were visually inspected to determine abuse deterrence following alcohol extraction, and also following subsequent filtration.

The purpose of this study is to perform and summarize the results of an alcohol extraction, filtration, and visual examination of the resulting extraction solution for different formulations. Formulations of CII narcotic drug products can be modified from their intended dosage form in order to receive immediate release of the full dose of the active pharmaceutical ingredient into the body. This is known as making the drug product "abusable." Formulation development has occurred which is intended to reduce the ability of patients to modify the products into this "abusable" form. Extrusion and compress-and-curing are two methods for manufacturing CII drug products. Both methods, when formulated appropriately, possess characteristics which reduce the ability of patients to modify the products into an "abusable" form (when compared to traditional methods).

Twin Screw extrusion can be described as mixing a blended formulation by using shear forces. The co-rotating screws create shear/frictional forces through material contact between the two screws and between the screws and barrel wall. The shear forces work on the material based on its viscosity (inter-particulate friction) to create a homogenous polymer melt. The heated barrels control the melt by maintaining constant temperatures in the various zones of the extruder as well as add additional heat to maintain energy in the process. This happens in a simultaneous continuous process while the material is transferred through the extruder. The polymer melt can then be pushed through a die to form a uniform extrudate. This differs from compress-and-curing which can be described as initially compressing (with force) the blended formulation and then curing (with heat) after the compression in a separate sequential process to produce a finished drug product. CII drug products which utilize each manufacturing method are currently commercially available. In some embodiments, the formulation of the present disclosure is formed by an extrusion process under sufficient shear stresses to impart strength and stability to the formulation. The formulation can be prepared using an extruder wherein shear forces, pressure, and heating are be applied together or separately in different zones of the extruder. In some embodiments, the formulation is prepared by reaching a melt flow temperature of the specific formulation in the extruder to assist in producing a uniform extrudate (i.e., localized uniformity). Compress-and-curing formulations are not similarly prepared.

Three principal methods of modifying CII drug products in order to make them "abusable" exist, namely cutting, grinding, and extraction. Cutting the dosage form can be performed in order to increase the surface area of the product prior to ingesting it in an effort to increase the rate of dissolution into the digestive tract. Cutting can also be used to increase the efficiency of extraction by breaking/removing the aqueous, non-ethanol soluble coating applied to many commercially available drugs. Cutting alone, however, is not sufficient to render a formulation abuseable. Readily available tools used for cutting are razor blades and common kitchen scissors. Grinding the dosage form is performed in order to decrease the particle size of the product in an effort to insufflate (snort) for immediate release into the blood vessels of the nasal passages. Additional abuse pathways exist which follow the grinding of the product. A readily available tool used for grinding is a commercially available coffee grinder. Extraction is performed in order to dissolve the active pharmaceutical ingredient of the dosage form into a liquid which can be filtered and subsequently swallowed, injected, or otherwise abused. A readily available tool used for extraction is high potency alcohol (i.e., ≥190 proof (95%)).

Color is one identifying characteristic of commercial drug products. Color can be applied to the dosage form in two ways: dye or coating. High potency alcohol (i.e., ≥190 proof (95%)) is one extraction solvent that can be used by abusers for APIs which are insoluble in water or in order to separate the API from other water soluble excipients. Dyes or coatings can potentially be used to alter the physical appearance of the extracted solution of drug product (i.e., turn the resulting solution a noticeable color).

In this study, 190 proof ethanol was utilized as an extraction solvent. A commercially available coffee filter was used to filter out any particulate matter of several drug products. The resulting solution was analyzed for physical appearance. The difference in physical appearance (if any) between drug products which are dyed or coated was evaluated.

Additionally, a non ADF drug product which utilizes traditional compression manufacturing methods (Roxicodone® 15mg, manufactured by Mallinckrodt, Inc.) was evaluated for comparison purposes.

Experimental: The samples tested include ADF Oxycodone 5mg and 30mg (Immediate Release) and ADF Oxycodone 10mg and 80mg (Extended Release) as described in the present disclosure, as well as Roxicodone® 15mg (Mallinckrodt, Inc.), Opana® ER 5mg (reformulated) (Endo Health Solutions); Opana® ER 40mg (reformulated) (Endo Health Solutions); Oxycontin® 10mg (reformulated) (Purdue Pharma); Oxycontin® 40mg (reformulated) (Purdue Pharma); Oxycontin® 60mg (reformulated) (Purdue Pharma); Oxycontin® 80mg (reformulated) (Purdue Pharma). A summary of all of the samples tested is provided in the table below.

**Table 11: List of Samples Tested**

| Dosage Units Descriptions | | | | | |
|---|---|---|---|---|---|
| Sample | Sample Name | Manufac. Process | Manufac. Process Color | API | Release Timeframe |
| 1 | Roxicodone® 15mg | Compression | Dye | Oxycodone | Immediate |
| 2 | IR 5mg | Extrusion | Dye | Oxycodone | Immediate |
| 3 | IR 30mg | Extrusion | Dye | Oxycodone | Immediate |
| 4 | ER 10mg | Extrusion | Dye | Oxycodone | Extended |
| 5 | ER 80mg | Extrusion | Dye | Oxycodone | Extended |
| 6 | Opana® ER 5mg | Extrusion | Coating | Oxymorphone | Extended |
| 7 | Opana® ER 40mg | Extrusion | Coating | Oxymorphone | Extended |
| 8 | Oxycontin® 10mg | Compress & Cure | Coating | Oxycodone | Controlled |
| 9 | Oxycontin® 40mg | Compress & Cure | Coating | Oxycodone | Controlled |
| 10 | Oxycontin® 60mg | Compress & Cure | Coating | Oxycodone | Controlled |
| 11 | Oxycontin® 80mg | Compress & Cure | Coating | Oxycodone | Controlled |

The formulations of the samples of the present disclosure tested, i.e., samples 2 - 5, are provided in the table below.

**Table 12: Formulations of Samples Tested**

| Component | Sample 2 | Sample 3 | Sample 4 | Sample 5 |
|---|---|---|---|---|
| Oxycodone HCl | 5.00% | 30.00% | 5.00% | 33.33% |
| PEO, 100K Daltons | 35.00% | 35.00% | 40.00% | 40.00% |
| Microcrystalline Cellulose PH101 | 22.25% | 12.25% | | |
| Lactose Monohydrate 316 | 21.00% | 11.65% | | |
| Hypromellose, K100M | | | 37.50% | 20.00% |
| PEG, 8K Daltons | 15.00% | 10.00% | 15.75% | 4.67% |
| Citric Acid | 1.00% | 1.00% | 1.00% | 1.00% |
| Dye | 0.75% | 0.10% | 0.75% | 1.00% |
| Total weight | 100 mg | 100 mg | 200 mg | 240 mg |
| Release characteristics | IR | IR | ER | ER |

In additional embodiments of the present disclosure, the amount of active substance in the formulation can range from about 0.50 Wt% to about 40 Wt%. Particularly, the amount of active substance in the formulation may range from about 1.0 Wt% to about 35 Wt%, or from about 5.0 Wt% to about 33 Wt%. In additional embodiments of the present disclosure, the amount of plasticizer (e.g., PEG) can range from about 0.25 Wt% and about 20 Wt% plasticizer.

For each sample, both whole and cut dosage units were tested. For whole dosage units, two (2) whole dosage units were placed in a 25mL Erlenmeyer flask containing 10mL of EtOH. For cut dosage units, all cut pieces of the dosage unit were placed in similar flasks. Cut dosage units were cut into about 8 pieces using diagonal pliers. Each flask was sealed with parafilm and shaken on a platform shaker for at least 10 hours at about 150rpm. The resulting solution was filtered through a coffee filter to remove any particulate matter. The filtered solution was collected in a 50mL Nessler color comparison tube. After 30 minutes, each sample tube was visually examined for color (if any), clarity/turbidity, and if any noticeable difference in filtered solution volume exists (i.e., a significant decrease from the original 10mL EtOH). The results for the whole and cut dosage units are provided in the two tables below.

**Table 13: Whole Dosage Unit Extraction Data**

| Visual Examination - Whole Dosage Units | | | | | |
|---|---|---|---|---|---|
| Sample | Sample Name | Color Change | Color Observed | Intensity | Notes (clarity/turbidity, volume change, etc.) |
| 1 | Roxicodone® 15mg | Yes | Yellow | Faint | Clear, no volume change |
| 2 | IR 5mg | Yes | Yellow | Dark | Clear, no volume change |
| 3 | IR 30mg | Yes | Blue | Medium | Clear, ~1mL volume decrease |
| 4 | ER 10mg | Yes | Blue | Dark | Clear, ~3mL volume decrease |
| 5 | ER 80mg | Yes | Green | Dark | Clear, ~4mL volume decrease |
| 6 | Opana® ER 5mg | No | None | None | Clear, no volume change |
| 7 | Opana® ER 40mg | Yes | Yellow | Faint | Clear, no volume change |
| 8 | Oxycontin® 10mg | Yes | White | Faint | Slightly turbid, no volume change |
| 9 | Oxycontin® 40mg | Yes | White | Faint | Slightly turbid, no volume change |
| 10 | Oxycontin® 60mg | Yes | Red | Faint | Slightly turbid, no volume change |
| 11 | Oxycontin® 80mg | Yes | Blue | Faint | Slightly turbid, no volume change |

**Table 14: Cut Dosage Unit Extraction Data**

| Visual Examination - Cut Dosage Units | | | | | |
|---|---|---|---|---|---|
| Sample | Sample Name | Color Change | Color Observed | Intensity | Notes (clarity/turbidity, volume change, etc.) |
| 1 | Roxicodone® 15mg | Yes | Yellow | Faint | Clear, no volume change |
| 2 | IR 5mg | Yes | Yellow | Dark | Clear, no volume change |
| 3 | IR 30mg | Yes | Blue | Medium | Clear, ~1mL volume decrease |
| 4 | ER 10mg | Yes | Blue | Dark | Clear, ~3mL volume decrease |
| 5 | ER 80mg | Yes | Green | Dark | Clear, ~4mL volume decrease |
| 6 | Opana® ER 5mg | No | None | None | Clear, ~1 mL volume decrease |
| 7 | Opana® ER 40mg | Yes | Yellow | Faint | Clear, ~1mL volume decrease |
| 8 | Oxycontin® 10mg | Yes | White | Faint | Slightly turbid, ~1mL volume decrease |
| 9 | Oxycontin® 40mg | Yes | White | Medium | Turbid, ~1mL volume decrease |
| 10 | Oxycontin® 60mg | Yes | Red | Medium | Turbid, ~2mL volume decrease |
| 11 | Oxycontin® 80mg | Yes | Blue | Faint | Turbid, slight volume change |

During filtration, samples passed through the filter at various rates. For example, samples 1 and 6-11 took approximately 20 seconds for the entire volume to completely pass through the coffee filter. Samples 2 and 3 took approximately 15 minutes for the entire volume to completely pass through the coffee filter. Samples 4 and 5 took approximately 60 minutes for the entire volume to completely pass through the coffee filter. After filtration, samples 2-5 were uniform in color after sitting for approximately 30 minutes, while samples 8-11 had significant sediment at the bottom of the comparison tubes. Samples 1, 6 and 7 had no noticeable sediment but were significantly less colored than the batches of the present disclosure.

Approximately 5mL of the filtrate from each cut dosage form sample was passed through a 25mm, 0.2$\mu$m PTFE Titan syringe filter (Scientific Resources, Inc. Cat No. 42225-PC, Lot 709029003054). Each resulting solution was then assigned a number according to a scale of 0-5, with 0 (zero) representing a sample with no color and 5 representing a sample with a dark, significant color, (0 - no color; 1 - faint; 2 - light; 3 - medium; 4 - brilliant; and 5 - dark). Samples with at least light color, including dark coloration, can deter potential abusers from injecting or ingesting the filtered extract (e.g., colors 2 and above, 3 and above, 4 and above, or 5). The table below shows the color number assignments for the syringe filtered cut dosage unit solutions.

**Table 15: Cut Dosage Unit Color Numbers**

| Visual Examination - Cut Dosage Units | | |
| --- | --- | --- |
| Sample | Sample Name | Color Number |
| 1 | Roxicodone® 15mg | 1 |
| 2 | IR 5mg | 5 |
| 3 | IR 30mg | 3 |
| 4 | ER 10mg | 5 |
| 5 | ER 80mg | 5 |
| 6 | Opana® ER 5mg | 0 |
| 7 | Opana® ER 40mg | 1 |
| 8 | Oxycontin® 10mg | 0 |
| 9 | Oxycontin® 40mg | 0 |
| 10 | Oxycontin® 60mg | 0 |
| 11 | Oxycontin® 80mg | 0 |

In some embodiments, the formulation of the present disclosure incorporates the dye throughout the entire dosage unit as opposed to incorporating the dye only in a coating. The dye can be water soluble, alcohol soluble or both. The dye can have a solubility in water, alcohol or both that is greater than about 0.01g/100 mL, about 0.1g/100 mL, about 1g/100 mL or about 10g/100 mL. Traditional drug formulation dyes are not soluble, or significantly soluble, in water, alcohol or both. They are often formulated into the coatings of the drug formulations. In some embodiment, the dyes are water soluble, alcohol soluble or both, and are dyes that are approved for, or considered acceptable, for oral administration. In some instances, the solubility of the dye in alcohol is important because of the potential for compounding effects of, and interactions associated with, consuming both alcohol and the extracted API.

The following table lists the relative solubility of exemplary components of a formulation. A number of different dyes are listed along with their solubility information taken from the various literature sources and tested experimentally (200 proof ethanol and filtered through a 0.22 micrometer PTFE filter).

**Table 16: General Solubility of Exemplary Components**

| Exemplary Components | Water Solubility | Alcohol Solubility (Literature) | Alcohol Solubility (tested) |
| --- | --- | --- | --- |
| Oxycodone HCl | Yes | Yes | N/A |
| Polyethylene Oxide | Yes | No | N/A |
| Polyethylene Glycol | Yes | Yes | N/A |
| Hydroxypropylmethylcellulose | Yes | No | N/A |
| Microcrystalline Cellulose | No | No | N/A |

(continued)

| Exemplary Components | Water Solubility | Alcohol Solubility (Literature) | Alcohol Solubility (tested) |
|---|---|---|---|
| Lactose Monohydrate | Yes | No | N/A |
| FD&C Blue #1 | **Yes** | **Yes** | N/A |
| FD&C Blue #2 | **Yes** | **Yes** | **Yes** |
| FD&C Yellow #5 | **Yes** | **Yes** | **Yes** |
| FD&C Yellow #6 | **Yes** | **Yes** | **Yes** |
| FD&C Red #40 | **Yes** | **Yes** | **Yes** |
| Lake Dyes | No | No | N/A |

The sediment observed at the bottom of the comparison tubes of the Oxycontin® batches (samples 8-11) is indicative of a suspension rather than a solution. Typically, suspensions can be centrifuged or filtered to obtain a more clear solution (and in some cases, a colorless solution). Conversely, solutions cannot be further centrifuged or filtered using a common household coffee filter or a readily available syringe filter to obtain a more clear solution because the dye is completely dissolved in the solution. Dyed formulations can provide an additional mechanism of abuse deterrence than coated formulations.

The amount of dye present in the formulation can be an amount that produces an extract or a filtered extract using water, alcohol or a combination of both with a color that is greater than 0, or greater than 1, or greater than 2, or greater than 3 or greater than 4 on the visual scale disclosed, or similar scale. The amount of dye can vary depending on the formulation and components present. In some embodiments, the formulation can contain at least 0.1% dye, at least 0.2% dye, at least 0.3% dye, at least 0.4% dye, at least 0.5% dye, at least 0.6% dye, at least 0.7% dye, at least 0.8% dye, at least 0.9% dye, at least 1.0% dye, at least 1.5% dye, at least 2.0%, or any range of these values (e.g., between about 0.1% and about 1.0% dye).

It was also observed that a volume change occurred (~3-4mL decrease) for samples 4 and 5 following extended filtration time. Certain excipients (e.g., hydroxypropylmethylcellulose) can cause the resulting solution to become too viscous to fully pass through a coffee filter. Additional abuse deterrence (e.g., extended extraction time and volume loss) can be obtained by formulations including hydroxypropylmethylcellulose, or equivalents.

**Additional Exemplary Formulations**

Additional exemplary formulations of the present disclosure are provided in the tables below.

**Table 17: Additional Exemplary Formulations**

| Component | 15 | 15 | 20 | 30 | 40 | 60 |
|---|---|---|---|---|---|---|
| Oxycodone HCl | 15.00% | 7.50% | 10.00% | 15.00% | 20.00% | 30.00% |
| PEO, 100K Daltons | 35.00% | 40.00% | 40.00% | 40.00% | 40.00% | 40.00% |
| Microcrystalline Cellulose PH 101 | 18.75% | | | | | |
| Lactose Monohydrate 316 | 17.65% | | | | | |
| Hypromellose, K100M | | 33.00% | 31.00% | 29.00% | 29.00% | 28.00% |
| PEG, 8K Daltons | 12.50% | 17.50% | 17.85% | 14.60% | 9.25% | 0.25% |
| Citric Acid | 1.00% | 1.00% | 1.00% | 1.00% | 1.00% | 1.00% |
| Dye | 0.10% | 1.00% | 0.15% | 0.40% | 0.75% | 0.75% |
| | | | | | | |
| Total weight | 100 mg | 200 mg | 200 mg | 200 mg | 200 mg | 200 mg |
| Release characteristics | IR | ER | ER | ER | ER | ER |

**Table 18: Additional Exemplary Formulations**

| Component | | | | |
|---|---|---|---|---|
| Oxycodone HCl | 4.0 - 6.0% | 28.0 - 32.0% | 4.0 - 6.0% | 32.0 - 35.0% |
| PEO, 100K Daltons | 33.0 - 37.0% | 33.0 - 37.0% | 38.0 - 42.0% | 38.0 - 42.0% |
| Microcrystalline Cellulose PH 101 | 21.0 - 24.0% or 15.0 - 35.0% | 11.0 - 14.0% or 10.0 - 25.0% | | |
| Lactose Monohydrate 316 | 19.0 - 23.0% or 15.0 - 35.0% | 10.0 - 15.0% or 10.0 - 25.0% | | |
| Hypromellose, K100M | | | 36.0 - 39.0% | 18.0 - 22.0% |
| PEG, 8K Daltons | 13.0 - 17.0% | 8.0 - 12.0% | 14.0 - 17.0% | 4.0 - 6.0% |
| Citric Acid | 0.8 - 1.2% | 0.8 - 1.2% | 0.8 - 1.2% | 0.8 - 1.2% |
| Dye | 0.6 - 0.9% or 0.5 - 1.0% | 0.05 - 0.2% or 0.05 - 0.5% | 0.6 - 0.9% or 0.5 - 1.0% | 0.8 - 1.2% or 0.5 - 1.5% |
| Release characteristics | IR | IR | ER | ER |

**Table 19: Additional Exemplary Formulations**

| Component | | | | | | |
|---|---|---|---|---|---|---|
| Oxycodone HCl | 13.0-17.0% | 6.0-9.0% | 8.0-12.0% | 13.0-17.0% | 18.0-22.0% | 28.0-32.0% |
| PEO, 100K Daltons | 33.0-37.0% | 38.0-42.0% | 38.0-42.0% | 38.0-42.0% | 38.0-42.0% | 38.0-42.0% |
| Microcrystalline Cellulose PH 101 | 17.0-20.0% or 15.0-35.0% | | | | | |
| Lactose Monohydrate 316 | 16.0-19.0% or 15.0-35.0% | | | | | |
| Hypromellose, K100M | | 31.0-35.0% | 29.0-33.0% | 27.0-31.0% | 27.0-31.0% | 26.0-30.0% |
| PEG, 8K Daltons | 11.0-14.0% | 16.0-19.0% | 16.0-19.0% | 13.0-16.0% | 8.0-11.0% | 0.2-0.3% |
| Citric Acid | 0.8-1.2% | 0.8-1.2% | 0.8-1.2% | 0.8-1.2% | 0.8-1.2% | 0.8-1.2% |
| Dye | 0.05-0.2% or 0.05-0.5% | 0.8-1.2% or 0.75-1.25% | 0.1-0.3% or 0.1-0.5% | 0.3-0.5% or 0.3-0.8% | 0.6 - 0.9% or 0.5 - 1.0% | 0.6-0.9% or 0.5 - 1.0% |
| Release characteristics | IR | ER | ER | ER | ER | ER |

**Cutting Force Example**

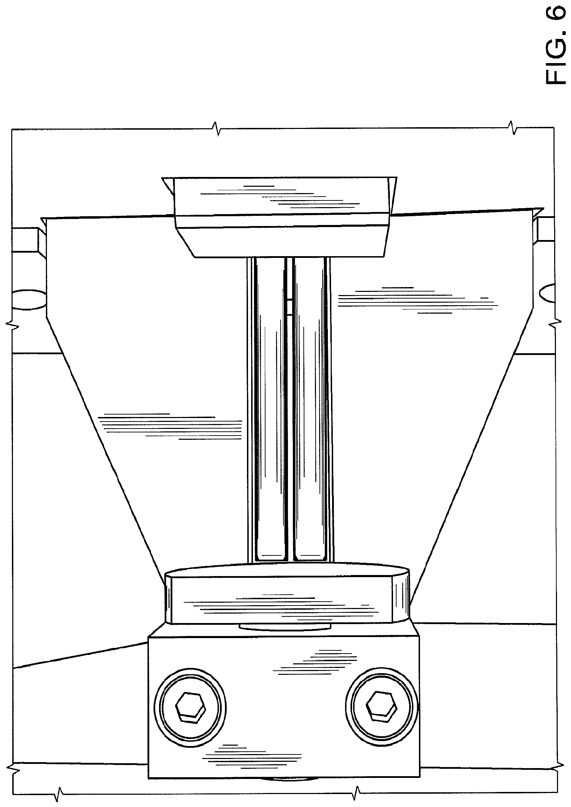

The existing methodology used to evaluate abuse deterrence in regard to the cutting or breaking of a dosage form is based on the USP's "tablet breaking force" test. This test defines "tablet breaking force" as the force required to cause tablets to fail (i.e., break) in a specific plane. The USP describes the test as follows "[t]he tablets are generally placed between two platens, one of which moves to apply sufficient force to the tablet to cause fracture. The platens should be parallel. Their faces should be polished smooth and precision-ground perpendicularly to the direction of movement. Perpendicularity must be preserved during platen movement, and the mechanism should be free of any bending or torsion displacements as the load is applied. The contact faces must be larger than the area of contact with the tablet." Figure 6 shows equipment capable of executing traditional "tablet breaking force" analysis.

The USP further explains the applications of tablet breaking force and why it is utilized in the industry. "Tablets must be able to withstand the rigors of handling and transportation experienced in the manufacturing plant, in the drug distribution system, and in the field at the hands of the end users (patients/consumers). Manufacturing processes such as coating, packaging, and printing can involve considerable stresses, which the tablets must be able to withstand. For these reasons, the mechanical strength of tablets is of considerable importance and is routinely measured." The intent of these applications is for traditional formulations which may be subjected to forces which could break the tablets (i.e., vigorous shaking in a tablet bottle). The intent is not to address abuse deterrence potential. Furthermore, this test is only applicable to and instructive to evaluate tablet formulations. The test is not applicable to or instructive to evaluate pill, or other formulations, prepared by extrusion methodologies.

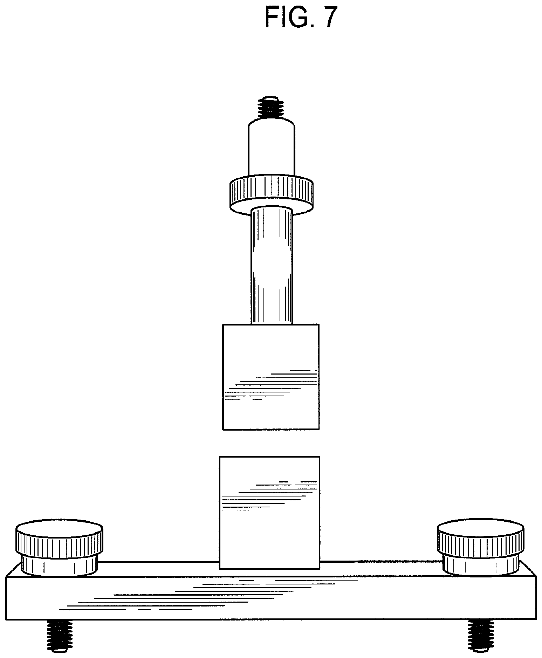

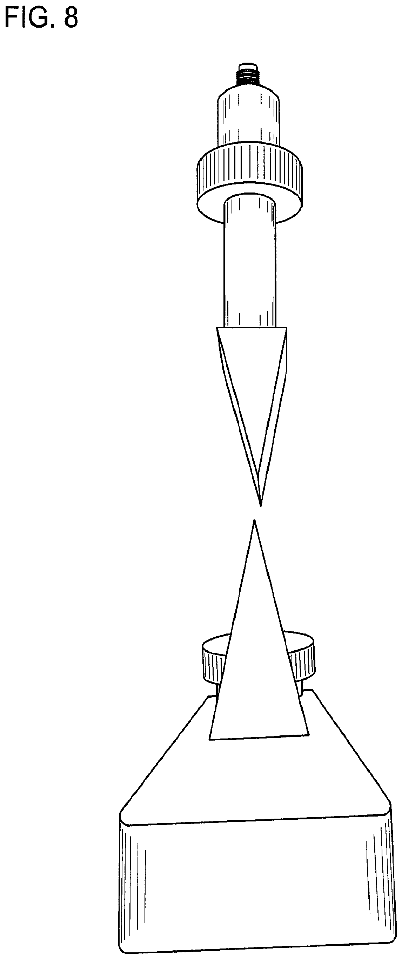

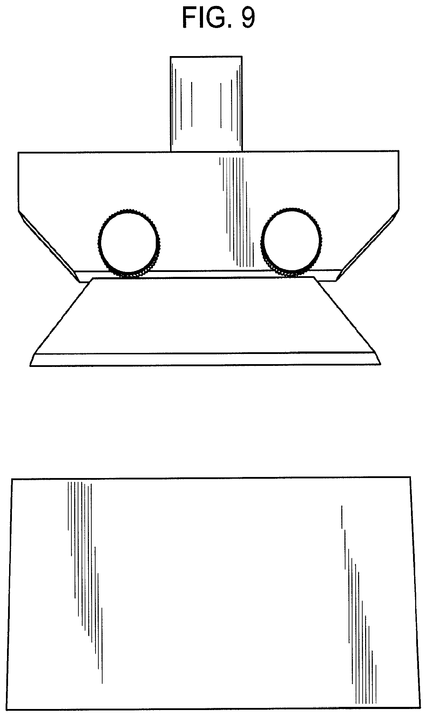

In formulations utilizing excipients such as polyethylene oxide, and using such excipients in an extrusion process, the parameter "tablet breaking force" does not apply. For example, the long molecular chain lengths of the PEO (e.g., 100,000 Daltons - 7,000,000 Daltons) cause the drug product (relative to other traditional drug products) to be flattened, but never actually "fail" (i.e., break) when applying "tablet breaking force" in the traditional sense. The traditional application of "tablet breaking force" needs to be modified to evaluate formulations containing malleable excipients (such as PEO) for the "cutting force" of the dosage form, specifically dosage forms which are intended to deter abuse. The modification of the traditional "tablet breaking force" test presented in this study consists of a change from the "platens" utilized to cause the dosage forms to "fail" (i.e., break), namely from contact faces "larger than the area of contact with the tablet" to sharp planes which mimic commonly used tools for abuse. Figures 7, 8 and 9 show reference attachments including a fracture wedge set (used to mimic common kitchen scissors, Figures 7 and 8 showing different views of the same set) and a razor blade (Figure 9).

The purpose of this study is to perform and summarize the cutting force needed to cut different formulations of CII narcotic drug products. Texture analysis is the mechanical testing of pharmaceutical products in order to measure their physical properties. A Texture Analyzer XT2i can perform testing of numerous physical properties of pharmaceutical products, including cutting force. The cutting force needed to cut several different formulations of CII narcotic drug products utilizing different attachments on a Texture Analyzer (TE37) was investigated. Multiple tools were utilized to cut drug products with the intent of abuse including two attachments which mimic readily available tools used for abuse (e.g., a razor blade and kitchen scissors). The cutting force for all evaluated drug products were evaluated with each attachment.

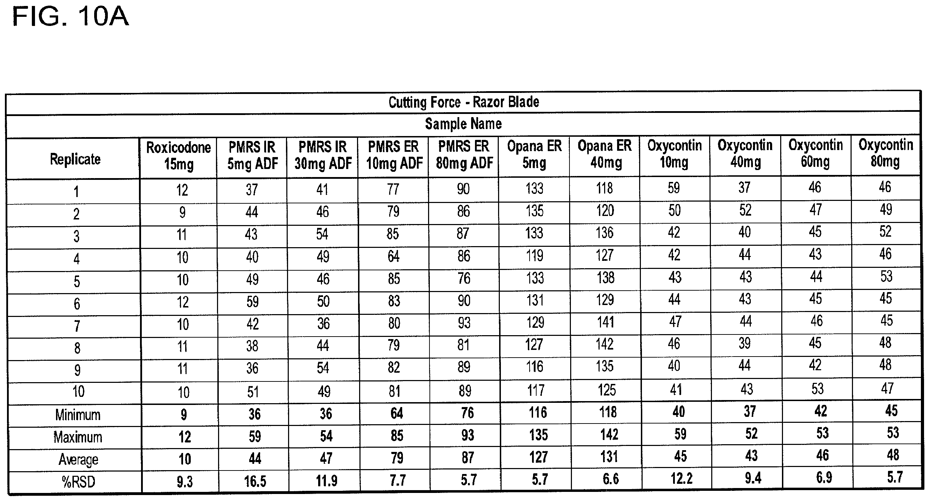

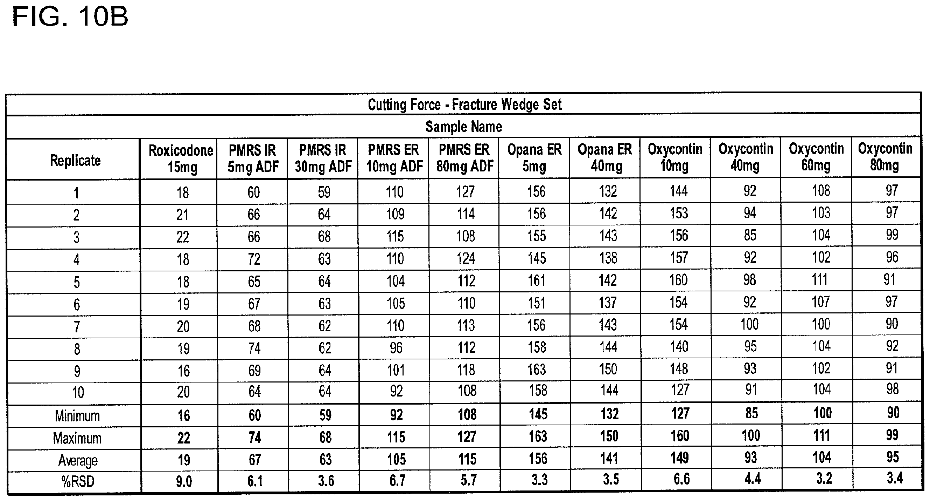

Experimental: The samples tested include those samples listed in Table A. The formulations of the samples of the present disclosure tested are listed in Table B. The Texture Analyzer (TE37), Model XT2i HR was operated at the following conditions: Pre Test Speed: 1mm/s; Test Speed: 0.25mm/s; Post Test Speed: 10mm/s; Distance: 99.9% (% Strain); Trigger Type: Auto (Force = 0.2N) and Break Detect: Off. A sample size of N=10 was used for each sample per cutting attachment. The cutting force results of the CII narcotic drug products utilizing both cutting attachments (razor blade and fracture wedge set) was determined. Figure 10 shows the cutting force data tables for the razor blade and the fracture wedge set.

The individual maximum cutting force needed to cut any tested CII narcotic drug products utilizing the razor blade was 142N (sample 7). The highest average cutting force needed to cut any tested CII narcotic drug products utilizing the razor blade was 131N (sample 7). The individual maximum cutting force needed to cut any tested CII narcotic drug products utilizing the fracture wedge set was 163N (sample 6). The highest average cutting force needed to cut any tested CII narcotic drug products utilizing the fracture wedge set was 156N (sample 6).

All of the tested CII narcotic drug products can indeed be cut, and therefore potentially be abused, with force which is substantially lower than what has been reported using the breaking strength test or equivalent (>500N, *See* U.S. Patent US 8,309,060) utilizing conventional means (i.e., common kitchen scissors or a razor blade). "Flattening" the tablets utilizing forces >500N (with traditional "tablet breaking force" definitions) does not address abuse deterrence potential in the tested CII narcotic drug products.

In one embodiment, the formulation of the present invention exhibits a cutting strength (i.e., force needed to cut the formulation) of greater than about 40 N, about 50 N, about 60 N, about 70 N, about 80 N, about 90 N, about 100 N, about 110 N, about 120 N, or about 130 N, or any range of these values (e.g., between about 40 N and about 120 N), as tested by either the Cutting Force - Razor Blade test or by the Cutting Force - Fracture Wedge Set test, or both.

Samples 4 of 5 of the present disclosure exhibit improved cutting strength compared to the compress-and-cure samples (i.e., samples 8-11). Samples prepared via a compress-and-cure procedure undergo dry mixing of the components only. These components are then compressed into a dosage form, and placing on a drying pan which applies heat to the dosage form. It is believed that compress-and-cure dosage forms are not melted or similarly liquefied to create significant homogeneity within the dosage form as compared to extrusion based procedures. The dosage formulations of the present invention are prepared by extrusion and, therefore, possess significant homogeneity as a result of the extrudate mixing within the extruder under melt flow conditions. The extrudate experiences high shear forces that produce the mechanical energy needed to ensure the required hardness and strength are achieved. The high shear forces can act on select components, for example PEO, to transform them into networks that exhibit increased strength and stability.

**Grinding Example**

The purpose of this study is to perform and summarize the grinding potential of different formulations of CII narcotic drug products. Texture analysis is the mechanical testing of pharmaceutical products in order to measure their physical properties. The Retsch Knife Mill GRINDOMIX GM200 (TE96) was utilized to mimic a commercially available coffee grinder (Mr. Coffee) in order to grind CII drug products into a particle size that is suitable for intranasal abuse (insufflation). A commercially available coffee grinder was also evaluated for comparison purposes. Particle size analysis was conducted utilizing an ATM L3P Sonic Sifter (TE47), utilizing a 500 micrometer ($\mu$m) particle size sieve (35 mesh). For the purposes of this study, any particle less than 500$\mu$m in diameter is considered suitable for intranasal abuse. It is generally accepted as an industry standard that any particle greater than 500$\mu$m in diameter cannot be sufficiently absorbed by the blood vessels in the nasal passages.

The Retsch Knife Mill GRINDOMIX GM200 utilizes a circular blade attachment to mimic commercially available coffee grinders. The GM200 has a top speed of 10,000 revolutions per minute (rpm), while commercially available coffee grinders have a top speed of approximately 20,000rpm (an approximate two-fold increase in speed when comparing the GM200 to a Mr. Coffee grinder). However, the approximate two-fold increase in blade diameter (118mm vs. 60mm, when comparing the GM200 to a Mr. Coffee grinder, respectively) compensates for the approximate twofold decrease in top speed via the inversely proportional relationship of the two variables. Further, the torque provided by the GM200 is significantly higher than the torque provided by a Mr. Coffee grinder (0.860Nm (Newton meters) of the GM200 vs. 0.062Nm of the Mr. Coffee grinder, respectively), which additionally illustrates the ability (or lack thereof) of the Mr. Coffee grinder to modify the drug products into a particle size suitable for intranasal abuse. The study evaluated the difference in particle sizes of several different formulations of CII narcotic drug products following modification (grinding) by the GM200 and Mr. Coffee grinder.

Additionally, a non ADF drug product which utilizes traditional compression manufacturing methods (Roxicodone® 15mg, manufactured by Mallinckrodt, Inc.) was evaluated for comparison purposes.

Experimental: The samples tested include those samples listed in Table A. The formulations of the samples of the present disclosure tested are listed in Table B. The following test equipment was used: Retsch Knife Mill GRINDOMIX GM200 (TE96), Coffee Grinder (Mr. Coffee), ATM L3P Sonic Sifter (TE47), 500$\mu$m sieve (35 mesh) and a Shimpo Instruments Tachometer (TE31). The following testing conditions were used: Analysis speed: 10,000rpm (GM200), 20,000rpm (Mr. Coffee); Analysis time: 30 seconds; Sieve Size: 500$\mu$m (35 mesh); Analysis time: 2 minutes (no pulse). Each sample was prepared in triplicate (N=3).

For each sample, three (3) dosage units were weighed and tested. The following conditions were used with the TE96: a 30 second analysis time and a speed of 10,000rpm. Both parameters were set prior to each analysis. The composite sample was transferred to a tared weigh boat and the weight of the sample was recorded. The following equation was used to calculate the % sample loss:

$$\text{Sample Loss (\%)} = 100 - \left( \frac{\text{Analyzed Sample (mg)}}{\text{Sample Weight (mg)}} \times 100 \right)$$

The weight of the 35 mesh sieve and sample pan was recorded. The testing apparatus was assembled with the 35 mesh sieve above the sample pan. The composite sample was transferred to the testing apparatus and analyzed utilizing the following parameters: 2 minute analysis time and no pulse. The analyzed 35 mesh sieve and sample pan were weighed. The % material remaining on the 35 mesh sieve ($\geq$ 500$\mu$m) and in the sample pan ($\leq$ 500$\mu$m) was calculated using the following equation:

$$\text{Percent on Sieve (\%)} = \frac{\text{Weight of Sample on Sieve (mg)}}{\text{Total Weight of Sample on Sieve (mg)}} \times 100$$

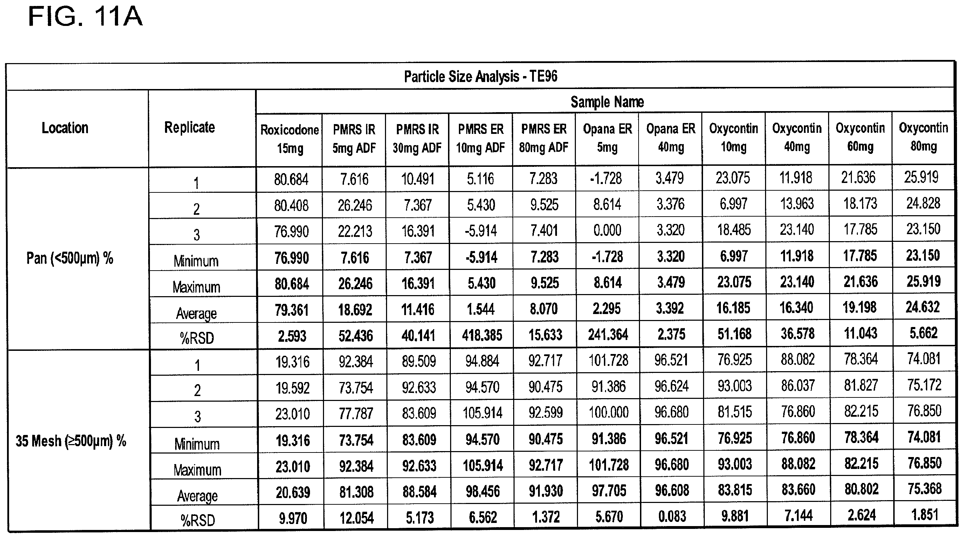

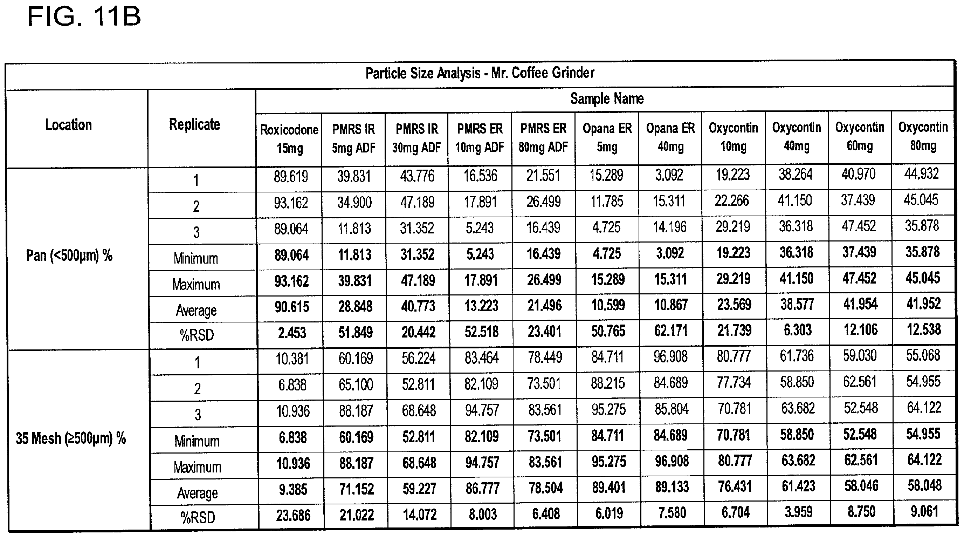

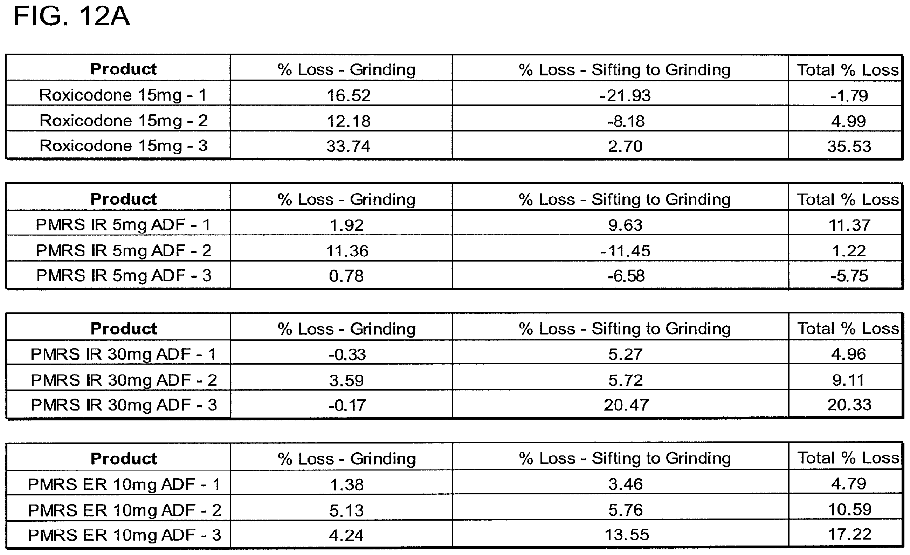

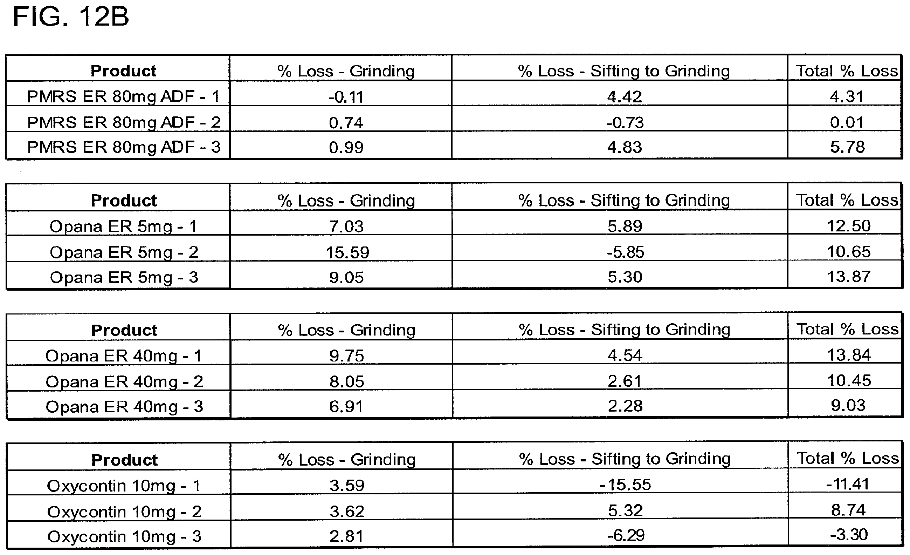

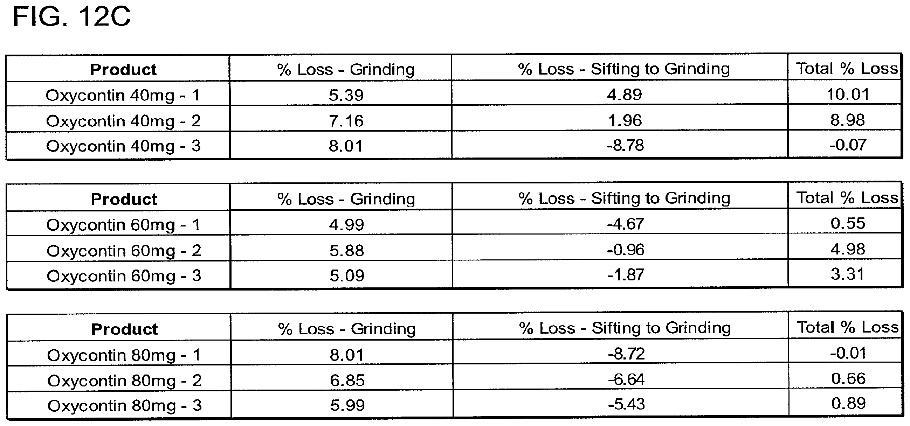

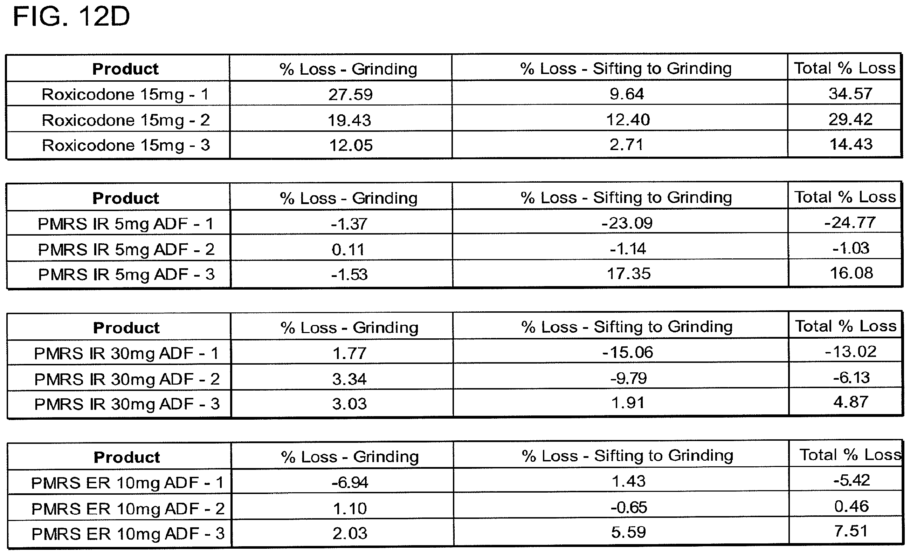

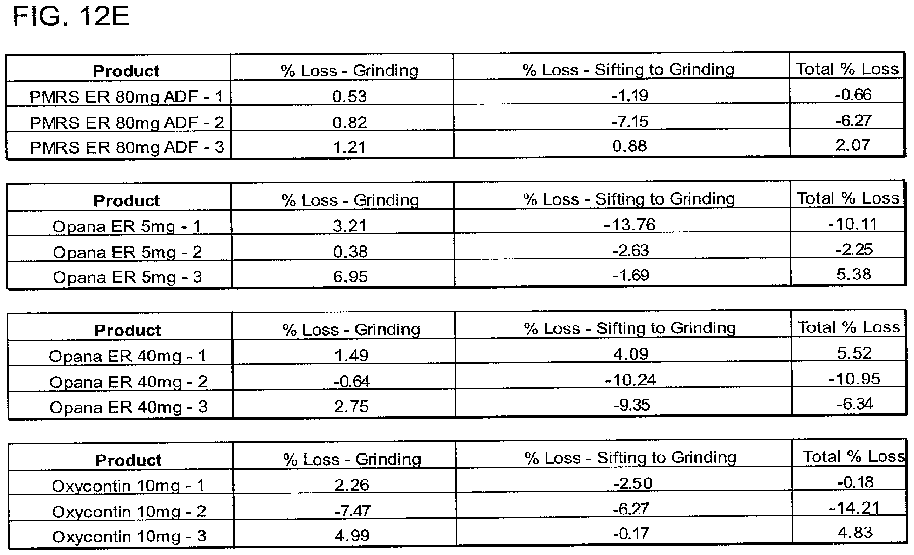

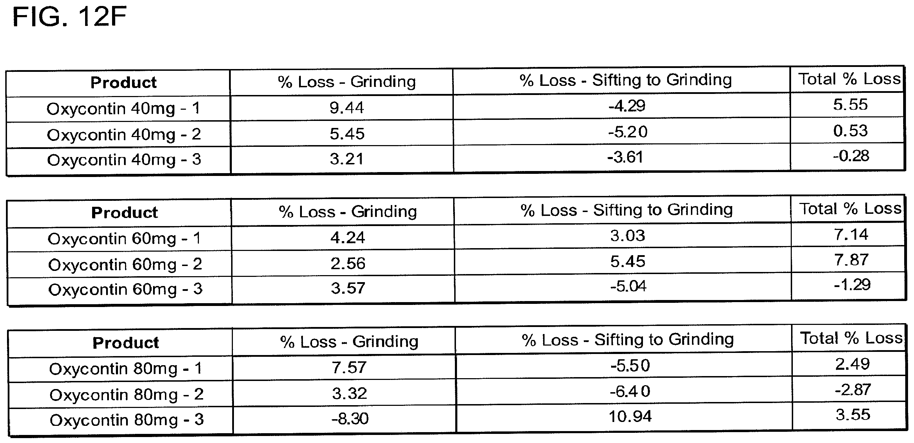

The procedure was repeated for the Mr. Coffee grinder in place of the TE96. The Mr. Coffee grinder has 1 operating speed (~20,000rpm). The particle size analysis and grinding results are shown in Figures 11 and 12.

The reference graph below is a representation of particle size results (% $\geq$500$\mu$m) when comparing the tested Immediate Release (IR) Roxicodone® batch vs. the formulations of the present disclosure (e.g., IR batches) utilizing both TE96 and the Mr. Coffee grinder.

* Roxicodone® batch % particles ≥500µm is statistically different (lower) than PMRS ADF IR batches.

The reference graph below is a representation of particle size results (% ≥500µm) when comparing the tested Extended Release (ER) CII narcotic drug products between manufacturers.

* Oxycontin® batch % particles ≥500µm is statistically different (lower) than PMRS ADF ER batches and Opana® batches when combining all dosages tested by each manufacturer.

The Roxicodone® batch provides statistically different (lower) amounts of particles ≥500µm than the formulations of the present disclosure (e.g., IR samples) following grinding and particle size analysis. Statistical significance was tested against a 95% confidence interval or a p-value of less than 0.05. Combined Oxycontin® batches provide statistically different (lower) amounts of particles ≥500µm than combined formulations of the present disclosure (e.g., ER samples and combined Opana® batches following grinding and particle size analysis as described in the protocol.

The results were combined per manufacturer, i.e. the present disclosure, Opana® ER batch results, and Oxycontin® results, and analyzed as groups. The combined Opana® batches provide statistically similar amounts of particles

≥500μm as the combined formulations of the present disclosure (e.g., ER samples) following grinding and particle size analysis.

**Example 4**

Abuse deterrent formulations were prepared containing both a dye and an alcohol purity decreasing agent. Table 20 shows an exemplary formulation.

**Table 20: Exemplary Abuse Deterrent Formulation Ranges**

| Components | Wt % | Wt % | Wt % |
|---|---|---|---|
| Active | 3.0 - 7.0 | 13.0 - 17.0 | 28.0 - 32.00 |
| Matrix | 33.0 - 37.0 | 33.0 - 37.0 | 33.0 - 37.0 |
| Plasticizer | 20.0 - 25.0 | 15.0 - 20.0 | 8.0 - 12.0 |
| Filler20.0 - 25.0 | 20.0 - 25.0 | 15.0 - 20.0 | 8.0 - 12.0 |
| Purity Decreasing Agent | 8.0 - 12.0 | 8.0 - 12.0 | 8.0 - 12.0 |
| Preservative | 0.5 - 2.0 | 0.5 - 2.0 | 0.5 - 2.0 |
| Dye | 2.0 - 6.0 | 2.0 - 6.0 | 2.0 - 6.0 |

**Table 21: Exemplary Abuse Deterrent Formulation for 100 mg IR pill**

| 100 mg IR Pill | 5 | 15 | 30 |
|---|---|---|---|
| Components | Wt % | Wt % | Wt % |
| Oxycodone Hydrochloride (active) | 5.0 | 15.0 | 30.0 |
| Sentry PolyOX WSR N-10 LEO, NF (matrix, e.g., PEO) | 35.0 | 35.0 | 35.0 |
| Carbowax Sentry Polyethylene Glycol 8000 Powder, NF (plasticizer) | 22.5 | 17.5 | 10.0 |
| Lactose Monohydrate 316L FastFlo (filler) | 22.5 | 17.5 | 10.0 |
| Calcium Acetate, USP (purity decreasing agent) | 10.0 | 10.0 | 10.0 |
| Citric Acid, Anhydrous Fine Ganular, USP (preservative) | 1.0 | 1.0 | 1.0 |
| Coloron Red Dye Blend (dye) | 4.0 | 4.0 | 4.0 |

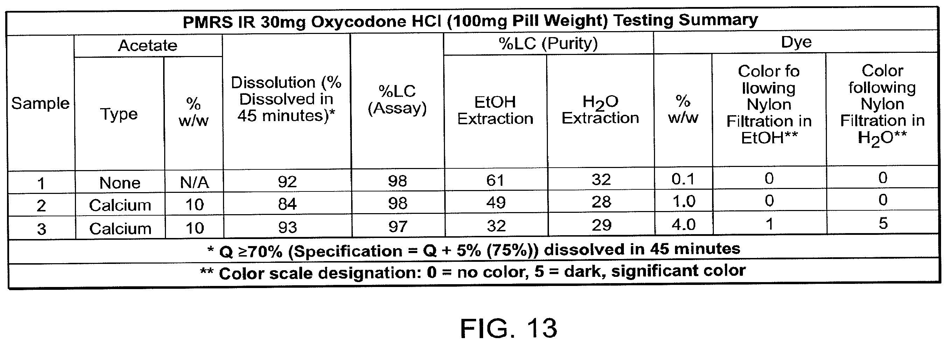

The exemplary formulation in Example 4 was tested for dissolution, purity before and after extraction, and evaluated for the effectiveness of the dye. The dissolution and dye tests are described above in the preceding examples. The extraction test is described below. Figure 13 shows the test results. The formulation passed dissolution testing. The formulation showed a decrease in purity after extraction in alcohol. It is noted that the formulation showed significant color after filtration with using about 4 wt% dye. Prior to filtering, the solutions were colored as a result of the dye being present in the formulation. In general, filtering the solution with a syringe filter did not substantially reduce the color of the filtrate. For nylon filters, however, a substantial reduction in color was observed. It is believed that nylon has an affinity to the dye used in the formulation. As a result, in some embodiments, a sufficient amount of dye is added to saturate any filter (e.g., a nylon filter) used to filter the solution and also to allow the filtrate to be colored (e.g., at least 1 wt% dye, at least 2 wt% dye, at least 3% dye, etc.)."

Extraction Procedure for ADF IR Extruded Pills

The extraction procedure involves testing the resultant purity of an extract using alcohol and / or water as the extraction solvent. Here, four whole ADF 30mg IR pills were placed in a 125mL Erlenmeyer flask. 40.0 mL of 190-proof ethanol (95%) was pipetted into the flask. The flask was sealed and allowed to shake overnight on a platform shaker (concentration of stock solution = 3 mg/mL oxycodone HCl).

This procedure was repeated utilizing water as the extraction solvent.

After approximately 12 hours, both flasks were removed from the platform shaker. A portion was filtered through a 0.45 µm nylon syringe filter into a 10 mL glass beaker. 5.0 mL of this solution was pipetted into a 150 mL beaker and heated on a hot plate at ~100 °C (theoretically 15 mg of oxycodone HCl powder in solution). The heat was continuously added until each solution evaporated. Both beakers were allowed to cool to room temperature and then scraped with a metal spatula. The resulting powder was weighed and subsequently prepared for chromatographic analysis.

Each sample was weight corrected and analyzed for assay purity of oxycodone HCl.

The assay purity is the percentage of oxycodone HCl in the extracted powder. As the assay purity of oxycodone HCl decreases the amount of extracted powder necessary to achieve higher oxycodone HCl content increases. Additionally, as the assay purity of oxycodone HCl decreases, the excipient load present in the extracted powder increases. Therefore, it can be concluded that drug products which provide, upon ethanol or water extraction, a lower assay purity of oxycodone HCl in extracted powder are abuse deterrent. This deterrence is evident in the following ways: cost effectiveness (i.e., the need for more powder to produce an equivalent amount of oxycodone HCl); time effectiveness (i.e., a powder of lower purity containing oxycodone HCl which cannot be separated from the excipients without complex, time-consuming chemistry procedures); and the potential for introducing a higher excipient load into the body.

While this disclosure has been particularly shown and described with reference to example embodiments thereof, it will be understood by those skilled in the art that various changes in form and details may be made therein without departing from the scope of the invention as defined by the appended claims.

**Claims**

1. An oral, immediate release, abuse deterrent dosage form comprising:

   (i) an active substance susceptible to abuse;
   (ii) a matrix agent, wherein the matrix agent is polyethylene oxide, has an average molecular weight between 50K Daltons and 150K Daltons, and is 45 wt% to 55 wt% of the dosage form; and
   (iii) a plasticizer, wherein the plasticizer comprises polyalkalene glycol and has an average molecular weight between 1K Daltons and 15K Daltons,

   wherein the active substance susceptible to abuse has an immediate release profile such that the amount of said active substance released from the dosage form upon exposure to a de-aerated water within 45 minutes, as measured according to USP Monograph performed on a dissolution apparatus utilizing USP <711> Apparatus II (Paddles) with 500 mL de-aerated water as media and a paddle speed of 50 rpm, is greater than or equal to 75%, and
   wherein the composition includes a physical barrier to reduce abuse, wherein the physical barrier in the dosage form has at least 50 wt% of particles with a particle size greater than 0.5 mm following physical or mechanical manipulation of the dosage form; and
   wherein the dosage form is a formed, uniform extrudate having a uniform blend of active, matrix agent and plasticizer, and is directly formed from an extrusion process.

2. The oral, immediate release, abuse deterrent dosage form of claim 1, wherein the active substance is Oxycodone HCl or Hydrocodone Bitartrate.

3. The oral, immediate release, abuse deterrent dosage form of any one of claims 1 to 2, wherein the dosage form contains 5 Wt% to 60 Wt% of plasticizer.

4. The oral, immediate release, abuse deterrent dosage form of any one of claims 1 to 3, further comprising a filler and/or a disintegrant and/or an alcohol purity decreasing agent and/or a FD&C dye.

5. The oral, immediate release, abuse deterrent dosage form of any one of claims 1 to 4, further comprising 0.1 Wt% to 2.0 Wt% of one or more preservatives.

6. The oral, immediate release, abuse deterrent dosage form of any one of claims 1 to 5, wherein less than or equal to 60 Wt% of the active substance is released from the dosage form at 20 minutes.

7. The oral, immediate release, abuse deterrent dosage form of any one of claims 1 to 6 comprising:

   (ii) 48 Wt% to 52 Wt% polyethylene oxide as a matrix agent, wherein the matrix agent has an average molecular weight between 90K and 110K Daltons; and
   (iii) 15 Wt% to 20 Wt% polyethylene glycol having an average molecular weight between 7K and 9K Daltons as the plasticizer.

8. A process for the production of an oral, immediate release, abuse deterrent dosage form containing at least one active substance susceptible to abuse according to any one of claims 1 to 7 comprising:

(i) processing a uniform blend of the at least one active substance susceptible to abuse, polyethylene oxide (PEO) as a matrix agent and polyalkalene glycol as a plasticizer by hot melt extrusion using an extruder to make an extrudate; and
(ii) forming the extrudate using a forming unit into the dosage form.

9. The process for the production of an oral, immediate release, abuse deterrent dosage form of claim 8,

(a) wherein the forming unit is a unit capable of forming the dosage form without prior cutting of the extrudate, and/or
(b) wherein the processing of the uniform blend is performed at a processing temperature and a processing pressure such that the matrix agent and plasticizer soften and the at least one active substance susceptible to abuse does not substantially degrade, and

(bi) wherein at least one temperature zone of the extruder has a processing temperature equal to or below 75 °C and/or
(bii) wherein at least one pressure zone of the extruder has a processing pressure equal to or above 5 Bar.

10. A process for the production of an oral, immediate release, abuse deterrent dosage form containing at least one active substance susceptible to abuse according to claim 8 comprising:

(i) combining the at least one active substance susceptible to abuse, polyethylene oxide (PEO) as a matrix agent and polyalkalene glycol as a plasticizer in a hopper to form a mixture;
(ii) blending the mixture in the hopper until the uniform blend is achieved;
(iii) monitoring the mixture during blending using a process analytical technique to determine when the uniform blend is achieved;
(iv) feeding the uniform blend into the extruder;
(v) processing the uniform blend by hot melt extrusion in the extruder to make the extrudate;
(vi) transferring the extrudate to the forming unit using a transfer unit capable of controlling the temperature, pressure, environment or shape of the extrudate;
(vii) forming the extrudate using the forming unit into the dosage form; and
(viii) determining the quality, volume and weight of the dosage form using an optical inspection technique.

11. A dosage form of any one of claims 1 to 7 for use in the treatment of pain.

12. A dosage form of claim 1, wherein the plasticizer is 5 wt% to 20 wt% of the dosage form.

13. A dosage form of claim 4, wherein said filler is 5 wt% to 40 wt% of the dosage form, optionally 20 wt% to 30 wt% of the dosage form.

**Patentansprüche**

1. Missbrauchssichere orale Dosierungsform mit unmittelbarer Freisetzung, die Folgendes umfasst:

(i) eine für Missbrauch anfällige aktive Substanz;
(ii) ein Matrixmittel, wobei das Matrixmittel Polyethylenoxid ist, ein mittleres Molekulargewicht zwischen 50 kDa und 150 kDa aufweist und 45 Gew.-% bis 55 Gew.-% der Dosierungsform ausmacht; und
(iii) einen Weichmacher, wobei der Weichmacher Polyalkylenglykol umfasst und ein mittleres Molekulargewicht zwischen 1K Dalton und 15K Dalton aufweist,

wobei die für Missbrauch anfällige aktive Substanz ein solches Profil der unmittelbaren Freisetzung aufweist, dass die Menge der aktiven Substanz, die nach der Aussetzung gegenüber entlüftetem Wasser innerhalb von 45 Minuten aus der Dosierungsform freigesetzt wird, laut der Messung gemäß USP Monograph, die auf einer Auflösungsvorrichtung unter Verwendung der USP<711> Vorrichtung II (Schaufelrührer) mit 500 ml entlüftetem Wasser als Medium und einer Rührgeschwindigkeit von 50 U/min durchgeführt wurde, ≥ 75 % ist und wobei die Zusammensetzung eine physische Barriere zur Verringerung von Missbrauch umfasst, wobei die physische Barriere in der Dosierungsform zumindest 50 Gew.-% von Teilchen mit einer Teilchengröße über 0,5 mm nach der physikalischen oder mechanischen Manipulation der Dosierungsform aufweist; und
wobei die Dosierungsform ein geformtes, homogenes Extrudat mit einer homogenen Mischung aus aktiver Substanz, Matrixmittel und Weichmacher ist und direkt aus einem Extrusionsverfahren geformt wird.

2. Missbrauchssichere orale Dosierungsform mit unmittelbarer Freisetzung nach Anspruch 1, wobei die aktive Substanz Oxycodon-HCl oder Hydrocodonbitartrat ist.

3. Missbrauchssichere orale Dosierungsform mit unmittelbarer Freisetzung nach einem der Ansprüche 1 bis 2, wobei die Dosierungsform 5 Gew.-% bis 60 Gew.-% Weichmacher enthält.

4. Missbrauchssichere orale Dosierungsform mit unmittelbarer Freisetzung nach einem der Ansprüche 1 bis 3, die weiters einen Füllstoff und/oder ein Sprengmittel und/oder ein Mittel zur Verringerung der Alkoholreinheit und/oder einen FD&C-Farbstoff umfasst.

5. Missbrauchssichere orale Dosierungsform mit unmittelbarer Freisetzung nach einem der Ansprüche 1 bis 4, die weiters 0,1 Gew.-% bis 2,0 Gew.-% eines oder mehrerer Konservierungsmittel umfasst.

6. Missbrauchssichere orale Dosierungsform mit unmittelbarer Freisetzung nach einem der Ansprüche 1 bis 5, wobei nach 20 Minuten weniger als oder 60 Gew.-% der aktiven Substanz aus der Dosierungsform freigesetzt sind.

7. Missbrauchssichere orale Dosierungsform mit unmittelbarer Freisetzung nach einem der Ansprüche 1 bis 6, das Folgendes umfasst:

   (ii) 48 Gew.-% bis 52 Gew.-% Polyethylenoxid als Matrixmittel, wobei das Matrixmittel ein mittleres Molekulargewicht zwischen 90 kDa und 110 kDa aufweist; und
   (ii) 15 Gew.-% bis 20 Gew.-% Polyethylenlykol mit einem mittleren Molekulargewicht zwischen 7 kDa und 9 kDa als Weichmacher.

8. Verfahren zur Herstellung einer missbrauchssicheren oralen Dosierungsform mit unmittelbarer Freisetzung, die zumindest eine für Missbrauch anfällige aktive Substanz enthält, nach einem der Ansprüche 1 bis 7, das Folgendes umfasst:

   (i) das Verarbeiten einer homogenen Mischung aus der zumindest einen für Missbrauch anfälligen aktiven Substanz, Polyethylenoxid (PEO) als Matrixmittel und Polyalkylenglykol als Weichmacher durch Heißschmelzextrusion unter Verwendung eines Extruders, um ein Extrudat zu erzeugen; und
   (ii) das Formen des Extrudats zu der Dosierungsform unter Verwendung einer Formeinheit.

9. Verfahren zur Herstellung einer missbrauchssicheren oralen Dosierungsform mit unmittelbarer Freisetzung nach Anspruch 8,

   (a) wobei die Formeinheit eine Einheit ist, die in der Lage ist, die Dosierungsform zu formen, ohne das Extrudat zuvor zu schneiden, und/oder
   (b) wobei das Verarbeiten der homogenen Mischung bei einer solchen Verarbeitungstemperatur und einem solchen Verarbeitungsdruck durchgeführt wird, dass das Matrixmittel und der Weichmacher weich werden und die zumindest eine für Missbrauch anfällige aktive Substanz im Wesentlichen nicht zersetzt wird, und

   (bi) wobei zumindest ein Temperaturbereich des Extruders eine Verarbeitungstemperatur ≤ 75 °C aufweist und/oder
   (bii) wobei zumindest ein Druckbereich des Extruders einen Verarbeitungsdruck ≥ 5 bar aufweist.

10. Verfahren zur Herstellung einer missbrauchssicheren oralen Dosierungsform mit unmittelbarer Freisetzung, die zumindest eine für Missbrauch anfällige aktive Substanz enthält, nach Anspruch 8, das Folgendes umfasst:

   (i) das Kombinieren von der zumindest einen für Missbrauch anfälligen aktiven Substanz, Polyethylenoxid (PEO) als Matrixmittel und Polyalkylenglykol als Weichmacher in einem Einfülltrichter, um ein Gemisch zu erzeugen;

(ii) das Mischen des Gemischs im Einfülltrichter, bis die homogene Mischung erreicht ist;

(iii) das Überwachen des Gemischs während des Mischens unter Verwendung eines Prozessanalyseverfahrens, um zu bestimmen, wann die homogene Mischung erreicht ist;

(iv) das Einspeisen der homogenen Mischung in den Extruder;

(v) das Verarbeiten der homogenen Mischung durch Heißschmelzextrusion im Extruder, um das Extrudat zu erzeugen;

(vi) das Überführen des Extrudats in die Formeinheit unter Verwendung einer Übertragungseinheit, die zur Steuerung von Temperatur, Druck, Umgebung oder Form des Extrudats in der Lage ist;

(vii) das Formen des Extrudats zu der Dosierungsform unter Verwendung der Formeinheit; und

(viii) das Bestimmen von Qualität, Volumen und Gewicht der Dosierungsform unter Verwendung eines optischen Überwachungsverfahrens.

11. Dosierungsform nach einem der Ansprüche 1 bis 7 zur Verwendung bei der Schmerzbehandlung.

12. Dosierungsform nach Anspruch 1, wobei der Weichmacher 5 Gew.-% bis 20 Gew.-% der Dosierungsform ausmacht.

13. Dosierungsform nach Anspruch 4, wobei der Füllstoff 5 Gew.-% bis 40 Gew.-% der Dosierungsform, gegebenenfalls 20 Gew.-% bis 30 Gew.-% der Dosierungsform, ausmacht.

## Revendications

1. Forme posologique orale anti-abus à libération immédiate, comprenant :

(i) une substance active susceptible de faire l'objet d'un abus ;

(ii) un agent de matrice, dans laquelle l'agent de matrice est du poly(oxyde d'éthylène), a un poids moléculaire moyen compris entre 50K Daltons et 150K Daltons, et représente 45 % en poids à 55 % en poids de la forme posologique ; et

(iii) un plastifiant, dans laquelle le plastifiant comprend du polyalkalène glycol et a un poids moléculaire moyen compris entre 1K Daltons et 15K Daltons,

dans laquelle la substance active susceptible de faire l'objet d'un abus a un profil de libération immédiate tel que la quantité de ladite substance active libérée à partir de la forme posologique lors d'une exposition à une eau désaérée pendant 45 minutes, telle que mesurée selon une monographie USP réalisée sur un appareil de dissolution utilisant USP <711> Apparatus II (pales) avec 500 ml d'eau désaérée comme fluide et une vitesse de pale de 50 t/min, est supérieure ou égale à 75 %, dans laquelle la composition comprend une barrière physique pour réduire les abus, dans laquelle la barrière physique dans la forme posologique a au moins 50 % en poids de particules ayant une taille de particule supérieure à 0,5 mm à la suite d'une manipulation physique ou mécanique de la forme posologique ; et

dans laquelle la forme posologique est un extrudat uniforme formé comportant un mélange uniforme d'agent de matrice actif et de plastifiant, et est directement formée à partir d'un procédé d'extrusion.

2. Forme posologique orale anti-abus à libération immédiate selon la revendication 1, dans laquelle la substance active est l'oxycodone HCl ou le bitartrate d'hydrocodone.

3. Forme posologique orale anti-abus à libération immédiate selon l'une quelconque des revendications 1 à 2, dans laquelle la forme posologique contient 5 % en poids à 60 % en poids de plastifiant.

4. Forme posologique orale anti-abus à libération immédiate selon l'une des quelconques revendications 1 à 3, comprenant en outre une charge et/ou un délitant et/ou un agent diminuant la pureté de l'alcool et/ou un colorant FD&C.

5. Forme posologique orale anti-abus à libération immédiate selon l'une des quelconques revendications 1 à 4, comprenant en outre 0,1 % en poids à 2,0 % en poids d'un ou plusieurs conservateurs.

6. Forme posologique orale anti-abus à libération immédiate selon l'une quelconque des revendications 1 à 5, dans laquelle 60 % en poids ou moins de la substance active sont libérés de la forme posologique à 20 minutes.

**7.** Forme posologique orale anti-abus à libération immédiate selon l'une des quelconques revendications 1 à 6, comprenant :

(ii) 48 % à 52 % en poids de poly(oxyde d'éthylène) en tant qu'agent de matrice, dans laquelle l'agent de matrice a un poids moléculaire moyen compris entre 90K et 110K Daltons ; et
(iii) 15 % en poids à 20 % en poids de polyéthylène glycol ayant un poids moléculaire moyen compris entre 7K et 9K Daltons en tant que plastifiant.

**8.** Procédé de production d'une forme posologique orale anti-abus à libération immédiate contenant au moins une substance active susceptible de faire l'objet d'un abus selon l'une des quelconques revendications 1 à 7, comprenant les étapes consistant à :

(i) traiter un mélange uniforme d'au moins une substance active susceptible de faire l'objet d'un abus, du polyoxyde d'éthylène (PEO) en tant qu'agent de matrice et du polyalkalène glycol en tant que plastifiant par extrusion à l'état fondu à l'aide d'une extrudeuse pour fabriquer un extrudat ; et
(ii) former l'extrudat en la forme posologique en utilisant une unité de formage.

**9.** Procédé de production d'une forme posologique orale anti-abus à libération immédiate selon la revendication 8,

(a) dans lequel l'unité de formage est une unité capable de former la forme posologique sans découpe préalable de l'extrudat, et/ou
(b) dans lequel le traitement du mélange uniforme est effectué à une température de traitement et à une pression de traitement telles que l'agent de matrice et le plastifiant se ramollissent et que la au moins une substance active susceptible de faire l'objet d'un abus ne se dégrade sensiblement pas, et

(bi) dans lequel au moins une zone de température de l'extrudeuse a une température de traitement égale ou inférieure à 75°C et/ou
(bii) dans lequel au moins une zone de pression de l'extrudeuse a une pression de traitement égale ou supérieure à 5 bar.

**10.** Procédé de production d'une forme posologique orale anti-abus à libération immédiate contenant au moins une substance active susceptible de faire l'objet d'un abus selon la revendication 8, comprenant les étapes consistant à :

(i) combiner la au moins une substance active susceptible de faire l'objet d'un abus, du poly(oxyde d'éthylène) (PEO) en tant qu'agent de matrice et du polyalkalène glycol en tant que plastifiant dans une trémie pour former un mélange ;
(ii) mélanger le mélange dans la trémie jusqu'à l'obtention du mélange uniforme ;
(iii) surveiller le mélange pendant le mélange en utilisant une technique analytique de processus pour déterminer quand le mélange uniforme est atteint ;
(iv) introduire le mélange uniforme dans l'extrudeuse ;
(v) traiter le mélange uniforme par extrusion à l'état fondu dans l'extrudeuse pour fabriquer l'extrudat ;
(vi) transférer l'extrudat vers l'unité de formage en utilisant une unité de transfert capable de commander la température, la pression, l'environnement ou la forme de l'extrudat ;
(vii) former l'extrudat en la forme posologique en utilisant l'unité de formage ; et
(viii) déterminer la qualité, le volume et le poids de la forme posologique à l'aide d'une technique d'inspection optique.

**11.** Forme posologique selon l'une des quelconques revendications 1 à 7, destinée à être utilisée dans le traitement de la douleur.

**12.** Forme posologique selon la revendication 1, dans laquelle le plastifiant représente 5 % en poids à 20 % en poids de la forme posologique.

**13.** Forme posologique selon la revendication 4, dans laquelle ladite charge représente 5 % en poids à 40 % en poids de la forme posologique, facultativement de 20 % en poids à 30 % en poids de la forme posologique.

FIG. 1

FIG. 2

FIG. 3

FIG. 4

EP 3 032 948 B1

FIG. 5

FIG. 6

# FIG. 7

FIG. 8

# FIG. 9

# FIG. 10A

| | Cutting Force - Razor Blade | | | | | | | | | | |
|---|---|---|---|---|---|---|---|---|---|---|---|
| | Sample Name | | | | | | | | | | |
| Replicate | Roxicodone 15mg | PMRS IR 5mg ADF | PMRS IR 30mg ADF | PMRS ER 10mg ADF | PMRS ER 80mg ADF | Opana ER 5mg | Opana ER 40mg | Oxycontin 10mg | Oxycontin 40mg | Oxycontin 60mg | Oxycontin 80mg |
| 1 | 12 | 37 | 41 | 77 | 90 | 133 | 118 | 59 | 37 | 46 | 46 |
| 2 | 9 | 44 | 46 | 79 | 86 | 135 | 120 | 50 | 52 | 47 | 49 |
| 3 | 11 | 43 | 54 | 85 | 87 | 133 | 136 | 42 | 40 | 45 | 52 |
| 4 | 10 | 40 | 49 | 64 | 86 | 119 | 127 | 42 | 44 | 43 | 46 |
| 5 | 10 | 49 | 46 | 85 | 76 | 133 | 138 | 43 | 43 | 44 | 53 |
| 6 | 12 | 59 | 50 | 83 | 90 | 131 | 129 | 44 | 43 | 45 | 45 |
| 7 | 10 | 42 | 36 | 80 | 93 | 129 | 141 | 47 | 44 | 46 | 45 |
| 8 | 11 | 38 | 44 | 79 | 81 | 127 | 142 | 46 | 39 | 45 | 48 |
| 9 | 11 | 36 | 54 | 82 | 89 | 116 | 135 | 40 | 44 | 42 | 48 |
| 10 | 10 | 51 | 49 | 81 | 89 | 117 | 125 | 41 | 43 | 53 | 47 |
| Minimum | 9 | 36 | 36 | 64 | 76 | 116 | 118 | 40 | 37 | 42 | 45 |
| Maximum | 12 | 59 | 54 | 85 | 93 | 135 | 142 | 59 | 52 | 53 | 53 |
| Average | 10 | 44 | 47 | 79 | 87 | 127 | 131 | 45 | 43 | 46 | 48 |
| %RSD | 9.3 | 16.5 | 11.9 | 7.7 | 5.7 | 5.7 | 6.6 | 12.2 | 9.4 | 6.9 | 5.7 |

## FIG. 10B

| | | | | | | Cutting Force - Fracture Wedge Set | | | | | |
|---|---|---|---|---|---|---|---|---|---|---|---|
| | | | | | | Sample Name | | | | | |
| Replicate | Roxicodone 15mg | PMRS IR 5mg ADF | PMRS IR 30mg ADF | PMRS ER 10mg ADF | PMRS ER 80mg ADF | Opana ER 5mg | Opana ER 40mg | Oxycontin 10mg | Oxycontin 40mg | Oxycontin 60mg | Oxycontin 80mg |
| 1 | 18 | 60 | 59 | 110 | 127 | 156 | 132 | 144 | 92 | 108 | 97 |
| 2 | 21 | 66 | 64 | 109 | 114 | 156 | 142 | 153 | 94 | 103 | 97 |
| 3 | 22 | 66 | 68 | 115 | 108 | 155 | 143 | 156 | 85 | 104 | 99 |
| 4 | 18 | 72 | 63 | 110 | 124 | 145 | 138 | 157 | 92 | 102 | 96 |
| 5 | 18 | 65 | 64 | 104 | 112 | 161 | 142 | 160 | 98 | 111 | 91 |
| 6 | 19 | 67 | 63 | 105 | 110 | 151 | 137 | 154 | 92 | 107 | 97 |
| 7 | 20 | 68 | 62 | 110 | 113 | 156 | 143 | 154 | 100 | 100 | 90 |
| 8 | 19 | 74 | 62 | 96 | 112 | 158 | 144 | 140 | 95 | 104 | 92 |
| 9 | 16 | 69 | 64 | 101 | 118 | 163 | 150 | 148 | 93 | 102 | 91 |
| 10 | 20 | 64 | 64 | 92 | 108 | 158 | 144 | 127 | 91 | 104 | 98 |
| Minimum | 16 | 60 | 59 | 92 | 108 | 145 | 132 | 127 | 85 | 100 | 90 |
| Maximum | 22 | 74 | 68 | 115 | 127 | 163 | 150 | 160 | 100 | 111 | 99 |
| Average | 19 | 67 | 63 | 105 | 115 | 156 | 141 | 149 | 93 | 104 | 95 |
| %RSD | 9.0 | 6.1 | 3.6 | 6.7 | 5.7 | 3.3 | 3.5 | 6.6 | 4.4 | 3.2 | 3.4 |

EP 3 032 948 B1

## FIG. 11A

| Particle Size Analysis - TE96 | | | | | | | | | | | | |
|---|---|---|---|---|---|---|---|---|---|---|---|---|
| | | Sample Name | | | | | | | | | | |
| Location | Replicate | Roxicodone 15mg | PMRS IR 5mg ADF | PMRS IR 30mg ADF | PMRS ER 10mg ADF | PMRS ER 80mg ADF | Opana ER 5mg | Opana ER 40mg | Oxycontin 10mg | Oxycontin 40mg | Oxycontin 60mg | Oxycontin 80mg |
| Pan (<500μm) % | 1 | 80.684 | 7.616 | 10.491 | 5.116 | 7.283 | -1.728 | 3.479 | 23.075 | 11.918 | 21.636 | 25.919 |
| | 2 | 80.408 | 26.246 | 7.367 | 5.430 | 9.525 | 8.614 | 3.376 | 6.997 | 13.963 | 18.173 | 24.828 |
| | 3 | 76.990 | 22.213 | 16.391 | -5.914 | 7.401 | 0.000 | 3.320 | 18.485 | 23.140 | 17.785 | 23.150 |
| | Minimum | 76.990 | 7.616 | 7.367 | -5.914 | 7.283 | -1.728 | 3.320 | 6.997 | 11.918 | 17.785 | 23.150 |
| | Maximum | 80.684 | 26.246 | 16.391 | 5.430 | 9.525 | 8.614 | 3.479 | 23.075 | 23.140 | 21.636 | 25.919 |
| | Average | 79.361 | 18.692 | 11.416 | 1.544 | 8.070 | 2.295 | 3.392 | 16.185 | 16.340 | 19.198 | 24.632 |
| | %RSD | 2.593 | 52.436 | 40.141 | 418.385 | 15.633 | 241.364 | 2.375 | 51.168 | 36.578 | 11.043 | 5.662 |
| 35 Mesh (≥500μm) % | 1 | 19.316 | 92.384 | 89.509 | 94.884 | 92.717 | 101.728 | 96.521 | 76.925 | 88.082 | 78.364 | 74.081 |
| | 2 | 19.592 | 73.754 | 92.633 | 94.570 | 90.475 | 91.386 | 96.624 | 93.003 | 86.037 | 81.827 | 75.172 |
| | 3 | 23.010 | 77.787 | 83.609 | 105.914 | 92.599 | 100.000 | 96.680 | 81.515 | 76.860 | 82.215 | 76.850 |
| | Minimum | 19.316 | 73.754 | 83.609 | 94.570 | 90.475 | 91.386 | 96.521 | 76.925 | 76.860 | 78.364 | 74.081 |
| | Maximum | 23.010 | 92.384 | 92.633 | 105.914 | 92.717 | 101.728 | 96.680 | 93.003 | 88.082 | 82.215 | 76.850 |
| | Average | 20.639 | 81.308 | 88.584 | 98.456 | 91.930 | 97.705 | 96.608 | 83.815 | 83.660 | 80.802 | 75.368 |
| | %RSD | 9.970 | 12.054 | 5.173 | 6.562 | 1.372 | 5.670 | 0.083 | 9.881 | 7.144 | 2.624 | 1.851 |

EP 3 032 948 B1

# FIG. 11B

| Particle Size Analysis - Mr. Coffee Grinder | | | | | | | | | | | | |
|---|---|---|---|---|---|---|---|---|---|---|---|---|
| | | Sample Name | | | | | | | | | | |
| Location | Replicate | Roxicodone 15mg | PMRS IR 5mg ADF | PMRS IR 30mg ADF | PMRS ER 10mg ADF | PMRS ER 80mg ADF | Opana ER 5mg | Opana ER 40mg | Oxycontin 10mg | Oxycontin 40mg | Oxycontin 60mg | Oxycontin 80mg |
| Pan (<500µm) % | 1 | 89.619 | 39.831 | 43.776 | 16.536 | 21.551 | 15.289 | 3.092 | 19.223 | 38.264 | 40.970 | 44.932 |
| | 2 | 93.162 | 34.900 | 47.189 | 17.891 | 26.499 | 11.785 | 15.311 | 22.266 | 41.150 | 37.439 | 45.045 |
| | 3 | 89.064 | 11.813 | 31.352 | 5.243 | 16.439 | 4.725 | 14.196 | 29.219 | 36.318 | 47.452 | 35.878 |
| | Minimum | 89.064 | 11.813 | 31.352 | 5.243 | 16.439 | 4.725 | 3.092 | 19.223 | 36.318 | 37.439 | 35.878 |
| | Maximum | 93.162 | 39.831 | 47.189 | 17.891 | 26.499 | 15.289 | 15.311 | 29.219 | 41.150 | 47.452 | 45.045 |
| | Average | 90.615 | 28.848 | 40.773 | 13.223 | 21.496 | 10.599 | 10.867 | 23.569 | 38.577 | 41.954 | 41.952 |
| | %RSD | 2.453 | 51.849 | 20.442 | 52.518 | 23.401 | 50.765 | 62.171 | 21.739 | 6.303 | 12.106 | 12.538 |
| 35 Mesh (≥500µm) % | 1 | 10.381 | 60.169 | 56.224 | 83.464 | 78.449 | 84.711 | 96.908 | 80.777 | 61.736 | 59.030 | 55.068 |
| | 2 | 6.838 | 65.100 | 52.811 | 82.109 | 73.501 | 88.215 | 84.689 | 77.734 | 58.850 | 62.561 | 54.955 |
| | 3 | 10.936 | 88.187 | 68.648 | 94.757 | 83.561 | 95.275 | 85.804 | 70.781 | 63.682 | 52.548 | 64.122 |
| | Minimum | 6.838 | 60.169 | 52.811 | 82.109 | 73.501 | 84.711 | 84.689 | 70.781 | 58.850 | 52.548 | 54.955 |
| | Maximum | 10.936 | 88.187 | 68.648 | 94.757 | 83.561 | 95.275 | 96.908 | 80.777 | 63.682 | 62.561 | 64.122 |
| | Average | 9.385 | 71.152 | 59.227 | 86.777 | 78.504 | 89.401 | 89.133 | 76.431 | 61.423 | 58.046 | 58.048 |
| | %RSD | 23.686 | 21.022 | 14.072 | 8.003 | 6.408 | 6.019 | 7.580 | 6.704 | 3.959 | 8.750 | 9.061 |

## FIG. 12A

| Product | % Loss - Grinding | % Loss - Sifting to Grinding | Total % Loss |
|---|---|---|---|
| Roxicodone 15mg - 1 | 16.52 | -21.93 | -1.79 |
| Roxicodone 15mg - 2 | 12.18 | -8.18 | 4.99 |
| Roxicodone 15mg - 3 | 33.74 | 2.70 | 35.53 |

| Product | % Loss - Grinding | % Loss - Sifting to Grinding | Total % Loss |
|---|---|---|---|
| PMRS IR 5mg ADF - 1 | 1.92 | 9.63 | 11.37 |
| PMRS IR 5mg ADF - 2 | 11.36 | -11.45 | 1.22 |
| PMRS IR 5mg ADF - 3 | 0.78 | -6.58 | -5.75 |

| Product | % Loss - Grinding | % Loss - Sifting to Grinding | Total % Loss |
|---|---|---|---|
| PMRS IR 30mg ADF - 1 | -0.33 | 5.27 | 4.96 |
| PMRS IR 30mg ADF - 2 | 3.59 | 5.72 | 9.11 |
| PMRS IR 30mg ADF - 3 | -0.17 | 20.47 | 20.33 |

| Product | % Loss - Grinding | % Loss - Sifting to Grinding | Total % Loss |
|---|---|---|---|
| PMRS ER 10mg ADF - 1 | 1.38 | 3.46 | 4.79 |
| PMRS ER 10mg ADF - 2 | 5.13 | 5.76 | 10.59 |
| PMRS ER 10mg ADF - 3 | 4.24 | 13.55 | 17.22 |

# FIG. 12B

| Product | % Loss - Grinding | % Loss - Sifting to Grinding | Total % Loss |
|---|---|---|---|
| PMRS ER 80mg ADF - 1 | -0.11 | 4.42 | 4.31 |
| PMRS ER 80mg ADF - 2 | 0.74 | -0.73 | 0.01 |
| PMRS ER 80mg ADF - 3 | 0.99 | 4.83 | 5.78 |

| Product | % Loss - Grinding | % Loss - Sifting to Grinding | Total % Loss |
|---|---|---|---|
| Opana ER 5mg - 1 | 7.03 | 5.89 | 12.50 |
| Opana ER 5mg - 2 | 15.59 | -5.85 | 10.65 |
| Opana ER 5mg - 3 | 9.05 | 5.30 | 13.87 |

| Product | % Loss - Grinding | % Loss - Sifting to Grinding | Total % Loss |
|---|---|---|---|
| Opana ER 40mg - 1 | 9.75 | 4.54 | 13.84 |
| Opana ER 40mg - 2 | 8.05 | 2.61 | 10.45 |
| Opana ER 40mg - 3 | 6.91 | 2.28 | 9.03 |

| Product | % Loss - Grinding | % Loss - Sifting to Grinding | Total % Loss |
|---|---|---|---|
| Oxycontin 10mg - 1 | 3.59 | -15.55 | -11.41 |
| Oxycontin 10mg - 2 | 3.62 | 5.32 | 8.74 |
| Oxycontin 10mg - 3 | 2.81 | -6.29 | -3.30 |

# FIG. 12C

| Product | % Loss - Grinding | % Loss - Sifting to Grinding | Total % Loss |
|---|---|---|---|
| Oxycontin 40mg - 1 | 5.39 | 4.89 | 10.01 |
| Oxycontin 40mg - 2 | 7.16 | 1.96 | 8.98 |
| Oxycontin 40mg - 3 | 8.01 | -8.78 | -0.07 |

| Product | % Loss - Grinding | % Loss - Sifting to Grinding | Total % Loss |
|---|---|---|---|
| Oxycontin 60mg - 1 | 4.99 | -4.67 | 0.55 |
| Oxycontin 60mg - 2 | 5.88 | -0.96 | 4.98 |
| Oxycontin 60mg - 3 | 5.09 | -1.87 | 3.31 |

| Product | % Loss - Grinding | % Loss - Sifting to Grinding | Total % Loss |
|---|---|---|---|
| Oxycontin 80mg - 1 | 8.01 | -8.72 | -0.01 |
| Oxycontin 80mg - 2 | 6.85 | -6.64 | 0.66 |
| Oxycontin 80mg - 3 | 5.99 | -5.43 | 0.89 |

# FIG. 12D

| Product | % Loss - Grinding | % Loss - Sifting to Grinding | Total % Loss |
|---|---|---|---|
| Roxicodone 15mg - 1 | 27.59 | 9.64 | 34.57 |
| Roxicodone 15mg - 2 | 19.43 | 12.40 | 29.42 |
| Roxicodone 15mg - 3 | 12.05 | 2.71 | 14.43 |

| Product | % Loss - Grinding | % Loss - Sifting to Grinding | Total % Loss |
|---|---|---|---|
| PMRS IR 5mg ADF - 1 | -1.37 | -23.09 | -24.77 |
| PMRS IR 5mg ADF - 2 | 0.11 | -1.14 | -1.03 |
| PMRS IR 5mg ADF - 3 | -1.53 | 17.35 | 16.08 |

| Product | % Loss - Grinding | % Loss - Sifting to Grinding | Total % Loss |
|---|---|---|---|
| PMRS IR 30mg ADF - 1 | 1.77 | -15.06 | -13.02 |
| PMRS IR 30mg ADF - 2 | 3.34 | -9.79 | -6.13 |
| PMRS IR 30mg ADF - 3 | 3.03 | 1.91 | 4.87 |

| Product | % Loss - Grinding | % Loss - Sifting to Grinding | Total % Loss |
|---|---|---|---|
| PMRS ER 10mg ADF - 1 | -6.94 | 1.43 | -5.42 |
| PMRS ER 10mg ADF - 2 | 1.10 | -0.65 | 0.46 |
| PMRS ER 10mg ADF - 3 | 2.03 | 5.59 | 7.51 |

## FIG. 12E

| Product | % Loss - Grinding | % Loss - Sifting to Grinding | Total % Loss |
|---|---|---|---|
| PMRS ER 80mg ADF - 1 | 0.53 | -1.19 | -0.66 |
| PMRS ER 80mg ADF - 2 | 0.82 | -7.15 | -6.27 |
| PMRS ER 80mg ADF - 3 | 1.21 | 0.88 | 2.07 |

| Product | % Loss - Grinding | % Loss - Sifting to Grinding | Total % Loss |
|---|---|---|---|
| Opana ER 5mg - 1 | 3.21 | -13.76 | -10.11 |
| Opana ER 5mg - 2 | 0.38 | -2.63 | -2.25 |
| Opana ER 5mg - 3 | 6.95 | -1.69 | 5.38 |

| Product | % Loss - Grinding | % Loss - Sifting to Grinding | Total % Loss |
|---|---|---|---|
| Opana ER 40mg - 1 | 1.49 | 4.09 | 5.52 |
| Opana ER 40mg - 2 | -0.64 | -10.24 | -10.95 |
| Opana ER 40mg - 3 | 2.75 | -9.35 | -6.34 |

| Product | % Loss - Grinding | % Loss - Sifting to Grinding | Total % Loss |
|---|---|---|---|
| Oxycontin 10mg - 1 | 2.26 | -2.50 | -0.18 |
| Oxycontin 10mg - 2 | -7.47 | -6.27 | -14.21 |
| Oxycontin 10mg - 3 | 4.99 | -0.17 | 4.83 |

# FIG. 12F

| Product | % Loss - Grinding | % Loss - Sifting to Grinding | Total % Loss |
|---|---|---|---|
| Oxycontin 40mg - 1 | 9.44 | -4.29 | 5.55 |
| Oxycontin 40mg - 2 | 5.45 | -5.20 | 0.53 |
| Oxycontin 40mg - 3 | 3.21 | -3.61 | -0.28 |

| Product | % Loss - Grinding | % Loss - Sifting to Grinding | Total % Loss |
|---|---|---|---|
| Oxycontin 60mg - 1 | 4.24 | 3.03 | 7.14 |
| Oxycontin 60mg - 2 | 2.56 | 5.45 | 7.87 |
| Oxycontin 60mg - 3 | 3.57 | -5.04 | -1.29 |

| Product | % Loss - Grinding | % Loss - Sifting to Grinding | Total % Loss |
|---|---|---|---|
| Oxycontin 80mg - 1 | 7.57 | -5.50 | 2.49 |
| Oxycontin 80mg - 2 | 3.32 | -6.40 | -2.87 |
| Oxycontin 80mg - 3 | -8.30 | 10.94 | 3.55 |

EP 3 032 948 B1

| PMRS IR 30mg Oxycodone HCl (100mg Pill Weight) Testing Summary | | | | | | | | | |
|---|---|---|---|---|---|---|---|---|---|
| Sample | Acetate | | Dissolution (% Dissolved in 45 minutes)* | %LC (Assay) | %LC (Purity) | | Dye | | |
| | Type | % w/w | | | EtOH Extraction | H$_2$O Extraction | % w/w | Color fo llowing Nylon Filtration in EtOH** | Color following Nylon Filtration in H$_2$O** |
| 1 | None | N/A | 92 | 98 | 61 | 32 | 0.1 | 0 | 0 |
| 2 | Calcium | 10 | 84 | 98 | 49 | 28 | 1.0 | 0 | 0 |
| 3 | Calcium | 10 | 93 | 97 | 32 | 29 | 4.0 | 1 | 5 |
| * Q ≥70% (Specification = Q + 5% (75%)) dissolved in 45 minutes | | | | | | | | | |
| ** Color scale designation: 0 = no color, 5 = dark, significant color | | | | | | | | | |

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61864926
- US 61980242 B
- US 8075872 B
- US 8383152 B
- US 20070190142 A
- US 20130028970 A1
- US 20120065220 A1
- US 20110159100 A1
- US 8309060 B

**Non-patent literature cited in the description**

- Guidance for Industry: Abuse-Deterrent Opioids - Evaluation and Labeling. U.S. Department of Health and Human Services, January 2013